# EUROPEAN PATENT SPECIFICATION

(11) **EP 0 794 966 B1**
(45) Date of publication and mention of the grant of the patent: **28.08.2002**
(21) Application number: 95937133.7
(22) Date of filing: 28.11.1995
(51) Int. Cl.: C07K 16/46, C07K 16/28, A61K 39/395

(54) **HUMANIZED ANTIBODIES TO CD38**
HUMANISIERTE ANTIKÖRPER GEGEN CD38
ANTICORPS HUMANISES DIRIGES CONTRE CD38

(30) Priority: 02.12.1994 GB 9424449
(43) Date of publication of application: 17.09.1997
(73) Proprietor: THE WELLCOME FOUNDATION LIMITED, Greenford, Middlesex UB6 0NN (GB)
(72) Inventor: ELLIS, Jonathan Henry, The Wellcome Research Lab., Beckenham Kent BR3 3BS (GB); LEWIS, Alan Peter, The Wellcome Research Lab., Beckenham Kent BR3 3BS (GB)
(74) Representative: Rees, Marion Lindsay
(86) International application number: GB9502777
(87) International publication number: WO96016990

(56) References cited:
- EP-A- 0 481 790
- WO-A-91/09967
- WO-A-94/09136
- WO-A-94/13805
- WO-A-94/17184
- JOURNAL OF MOLECULAR BIOLOGY, vol. 235, no. 1, 7 January 1994 LONDON, GB, pages 53-60, XP 000564648 A. CORTI ET AL. 'Idiotope determining regions of a mouse monoclonal antibody and its humanized versions.'
- JOURNAL OF MOLECULAR BIOLOGY, vol. 224, no. 2, 20 March 1992 LONDON, GB, pages 487-499, XP 000564649 J. FOOTE ET AL. 'Antibody framework residues affecting the conformation of the hypervariable loops.'
- PROCEEDINGS OF THE NATIONAL ACADEMY OF SCIENCES OF THE USA, vol. 89, no. 10, 15 May 1992 WASHINGTON, DC, USA, pages 4285-4289, XP 000275844 P. CARTER ET AL. 'Humanization of an anti-p185HER2 antibody for human cancer therapy.'
- THE JOURNAL OF IMMUNOLOGY, vol. 155, no. 2, 15 July 1995 BALTIMORE, MD, USA, pages 925-937, J. ELLIS ET AL. 'Engineered anti-CD38 monoclonal antibodies for immunotherapy of multiple myeloma.'
- IMMUNOLOGY, vol. 83, suppl. 1, 5 - 7 December 1994 OXFORD, GB, page 70 XP 000565931 J. ELLIS ET AL. 'Characterisation of a humanised monoclonal anti-CD38 antibody.'

## Description

The present invention relates to antibodies and in particular to humanised antibodies and their preparation.

Antibodies typically comprise two heavy chains linked together by disulphide bonds and two light chains. Each light chain is linked to a respective heavy chain by disulphide bonds. Each heavy chain has at one end a variable domain followed by a number of constant domains. Each light chain has a variable domain at one end and a constant domain at its other end. The light chain variable domain is aligned with the variable domain of the heavy chain. The light chain constant domain is aligned with the first constant domain of the heavy chain. The constant domains in the light and heavy chains are not involved directly in binding the antibody to antigen.

The variable domains of each pair of light and heavy chains form the antigen binding site. The variable domains on the light and heavy chains have the same general structure and each domain comprises a framework of four regions, whose sequences are relatively conserved, connected by three complementarity determining regions (CDRs: CDRL1, CDRL2, CDRL3, CDRH1, CDRH2 and CDRH3). The four framework regions largely adopt a beta-sheet conformation and the CDRs form loops connecting, and in some cases forming part of, the beta-sheet structure. The CDRs are held together in close proximity by the framework regions and, with the CDRs from the other domain, contribute to the formation of the antigen binding site. The four framework regions are therefore crucial in ensuring the correct positioning of the CDRs relative to each other and hence in antibody binding.

The importance of the interaction between the CDRs and the framework regions has become increasingly evident as more and more non-human antibodies have become humanised, such humanised antibodies comprising non-human CDRs within a human framework. Humanised antibodies, in contrast to non-human antibodies, say mouse or rat antibodies, elicit a negligible immune response when administered to a human.

The prior art discloses several ways of producing such humanised antibodies. Thus EP-A-0239400 describes splicing CDRs into a human framework. Briefly, the CDRs are derived from a non-human species such as a rat or mouse whilst the framework regions of the variable domains, and the constant domains, are derived from a human antibody. Specifically, a humanised anti-CD52 antibody is disclosed in EP-A-0328404.

EP-A-054951 describes another way of humanising an antibody by re-shaping a non-human antibody to make it more like a human antibody. Basically, it comprises taking a non-human variable domain, such as mouse or rat variable domain, and changing the residues in the framework region to correspond to residues of a human framework.

In both EP-A-0239400 and EP-A-054951 an altered antibody is produced in which the CDRs of the variable domain of the antibody are derived from a first non-human species and the framework regions and, if present, the or each constant domain of the antibody are derived from human.

In such humanised antibodies a number of residues of the human framework region appear to exert a critical influence on the affinity of antigen binding (for example Kettleborough et al, 1991, Prot.Eng. 4:773). Certain positions in the heavy chain framework regions, in particular, seem to be important in the retention of antigen-binding activity in a variety of altered antibodies. A number of investigators have reported the importance of residues at positions 67,69 and 71, within the heavy chain framework region. These residues form a beta-sheet in contact with the interior aspect of the CDRH2 loop: presumably mismatches at these positions distort the CDR shape. Also, residues at positions 91 and 94 appear to be important for correct CDRH3 conformation in many heavy chains ( for example Tempest et al, Biotechnology 9:266). Other positions likely to affect antigen-binding are residues 27, 30 and 94 in the heavy chain, and residue 49 and 71 in the light chain (numbering according to the Kabat system). Furthermore, in the heavy chain the importance of regions 66-73 and 27-30 has been recognised in the literature, with residues 66-73 lying in close contact with CDRH2.

In Corti A. *et al* ; J.Mol.Biol (1994) 235, pages 53-60, a humanised antibody is subjected to a number of subsitutions in the heavy chain framework regions to restore immunoreactivity to TNFα. These substitutions are with the original mouse amino acids. In one embodiment, 11 substitutions are made which include, *inter alia*, positions 29 and 78.
It has now been found that the residues at positions 29 and 78 of the framework region occupy a pocket which lies close to CDRH1 and affects antigen binding and that this undesirable effect can be obviated by using residues corresponding to those in the corresponding position of the framework region of the antibody from which the CDRs are derived.

Accordingly, the present invention is directed to a monoclonal antibody having donor CDRS of foreign origin and a recipient framework region having a sequence of human or primate origin wherein an original amino acid residue of the recipient framework region of the heavy chain is replaced by a replacement amino acid residue that is the same or similar to that in the corresponding position of the sequence of the corresponding framework region of the heavy chain from which the CDRs are derived characterised in that the replaced original amino acid residue consists of positions 29 and 78.

Typically, the original amino acid residues in positions 29 and/or 78 of the recipient framework region are larger than their corresponding residues in the framework region of the antibody from which the CDRs are derived. Examples of these larger residues include tyrosine, histidine, tryptophan and 2-phenylalanine. Examples of the smaller corresponding residues in the framework region of the antibody donating the CDRs include glycine, alanine, valine, serine and leucine. In accordance with the invention, the larger original residue in positions 29 and/or 78 of the recipient framework is replaced with a replacement amino acid residue that is either the same or similar to the corresponding smaller residue of the antibody which is donating the CDRs.

Although it is preferable for the replacement amino acid residue to be the same as the corresponding residue of the antibody which is donating the CDRs it can also be a similar amino acid residue provided the character with respect to size and preferably also hydrophobicity and charge is essentially the same i.e. conserved. For example, if the residue of the antibody which is donating the CDRs has a valine in position 29 and/or 78, then instead of having a replacement amino acid residue in the recipient framework which is also valine, one could, for example, use alanine instead since alanine is of equivalent charge, size and hydrophobicity to valine and thus similar. The use of a similar amino acid in place of the exact same amino acid is a practice which is well established in the art and known as conservative substitution.

By way of example, in a mouse heavy chain framework, side chains of Leu-29 and Val-78 would pack together in a small pocket close to CDRH1 whilst in the corresponding human heavy chain framework, such as for example NEW, which otherwise bears close homology to the mouse framework, the analogous positions are occupied by two Phe residues. The large aromatic side-chains appear to be too bulky to pack in the same fashion as in the mouse antibody and so alter the disposition of neighbouring surface residues resulting in a different conformation of CDRH1 in a humanised antibody. Substituting either Phe residue by the smaller murine residue partially relieves this effect allowing antigen binding. Full affinity is generally restored by replacement of both residues. It is therefore preferred that amino acids in both positions 29 and 78 are replaced.

In accordance with the invention, the replacement amino acid residues fit into the pocket without causing distortion of, for example, the CDRH1 conformation.

Preferably, the framework of the antibody heavy chain is homologous to the corresponding framework of the human antibody NEW (Saul *et al*, J. Biol. Chem. 253:585-597, 1978). The final residue of framework 1 in this case is suitably Ser or Thr, preferably Ser. This residue is at position 30 (Kabat *et al,* 1987). Preferably the framework of the antibody light chain is homologous to the variable domain framework of the protein REI (Epp *et al*, Eur. J. Biochem., 45:513-524, 1974).

Particular examples of murine heavy chains in which residues 29 and 78 pack together in a small pocket close to CDRH1 are those in Kabat groups IB and IIC.

By contrast, other examples of human heavy chains which have bulky residues in positions 29 and 78 in the framework region are LES-C, T52, Ab44, HIgI and NEW, as listed in Kabat.

Species other than the mouse that may have residues of a small size in positions 29 and 78 are for example, the rat, rabbit and hamster.

All amino acid residue positions referred to herein employ the Kabat numbering system.

An antibody according to the invention may be produced by a method including the steps of:
(i) obtaining the sequence of a donor heavy chain;
(ii) selecting a recipient human or primate framework by best-fit homology method;
(iii) replacing the amino acid residue in position 29 or 78 of the sequence of the recipient framework region of the heavy chain by an amino acid that is the same or similar to that in the corresponding position of the sequence of the corresponding framework region of the antibody from which the CDRs are derived.

The antibody heavy chain may be co-expressed with a complementary antibody light chain. At least the framework regions of the variable domain and the or each constant domain of the complementary chain generally are derived from the primate or human recipient. Preferably the CDRs of both chains are derived from the same selected antibody.

The antibody preferably has the structure of a natural antibody or a fragment thereof. The term antibody may therefore comprise a complete antibody, a (Fab')₂ fragment, a Fab fragment, Fv fragment, Fd fragment, SFv, a light chain dimer or a heavy chain and derivatives thereof. The antibody may be an IgG such as an IgG1, IgG2, IgG3 or IgG4, IgM, IgA, IgE or IgD. Furthermore, the antibody may comprise modifications of all classes e.g. IgG dimers, Fc mutants that no longer bind Fc receptors or mediate Clq binding (blocking antibodies). The antibody may also be a chimeric antibody of the type described in WO 86/01533) which comprises an antigen binding region and a non-immunoglobulin region. The antigen binding region is an antibody light chain variable domain or heavy chain variable domain. Typically, the antigen binding region comprises both light and heavy chain variable domains. The non-immunoglobulin region is fused at its C-terminus to the antigen binding region. The non-immunoglobulin region is typically a non-immunoglobulin protein and may be an enzyme, a toxin or a protein having known binding specificity. The two regions of the chimeric antibody may be connected via a cleavable linker sequence.

The invention is preferably employed to humanise an antibody, for example, an antibody of rat, rabbit, hamster or mouse origin. The framework regions and constant domains of the humanised antibody are therefore of human or primate origin whilst the CDRs of the light and/or heavy chain of the antibody are for example, rat or mouse CDRs. The antibody may be a human or primate IgG such as IgG1, IgG2, IgG3, IgG4; IgM; IgA; IgE or IgD in which the CDRs are of rat or mouse origin.

The antibody from which the donor CDRs are derived is typically an antibody of a selected specificity. In order to ensure that this specificity is retained, either the variable domain framework regions of the antibody are re-shaped to correspond to variable domain framework regions of a human or primate antibody or the CDRs are grafted onto the closest human or primate framework regions. Either way, the resulting antibody preferably comprises non-human CDRs and human or primate framework regions that are homologous with the corresponding framework regions of the antibody from which the CDRs are derived. Preferably there is a homology of at least 50% between the two variable domains.

There are four general steps to produce a humanised antibody. These are:
(1) determining the nucleotide and predicted amino acid sequence of the light and heavy chain variable domains of the antibody from which the CDRs are derived;
(2) deciding which human or primate antibody framework region to use;
(3) the actual grafting or re-shaping methodologies/techniques; and
(4) the transfection and expression of the grafted or re-shaped antibody.

These four steps are explained below.

### Step 1: Determining the nucleotide and predicted amino acid sequence of the antibody light and heavy chain variable domains

To humanise an antibody the amino acid sequence of the non-human antibody's (donor antibody's) heavy and light chain variable domains needs to be known. The sequence of the constant domains is irrelevant. The simplest method of determining an antibody's variable domain amino acid sequence is from cloned cDNA encoding the heavy and light chain variable domain.

There are two general methods for cloning a given antibody's heavy and light chain variable domain cDNAs: (1) via a conventional cDNA library, or (2) via the polymerase chain reaction (PCR). Both of these methods are widely known. Given the nucleotide sequence of the cDNAs, it is a simple matter to translate this information into the predicted amino acid sequence of the antibody variable domains.

### Step 2: Designing the humanised antibody

There are several factors to consider in deciding which human antibody (recipient antibody) sequence to use during humanisation. The humanisation of light and heavy chains are considered independently of one another, but the reasoning is basically the same.

This selection process is based on the following rationale: A given antibody's antigen specificity and affinity is primarily determined by the amino acid sequence of the variable region CDRs. Variable domain framework residues have little or no direct contribution. The primary function of the framework regions is to hold the CDRs in their proper spacial orientation to recognise the antigen. Thus the substitution of rodent CDRs into a human variable domain framework is most likely to result in retention of the correct spacial orientation if the human variable domain is highly homologous to the rodent variable domain from which the CDRs were derived. A human variable domain should preferably be chosen therefore that is highly homologous to the rodent variable domain(s).

A suitable human antibody variable domain sequence can be selected as follows:
(i) Using a computer program, search all available protein (and DNA) databases for those human antibody variable domain sequences that are most homologous, for example, to the rodent antibody variable domains. This can be easily accomplished with a program called FASTA but other suitable programs are available. The output of the program is a list of sequences most homologous to the rodent antibody, the percent homology to each sequence, and an alignment of each sequence to the rodent sequence. This is done independently for both the heavy and light chain variable domain sequences. The above analyses are more easily accomplished if customised sub-databases are first created that only include human immunoglobulin sequences. This has two benefits. First, the actual computational time is greatly reduced because analyses are restricted to only those sequences of interest rather than all the sequences in the databases. The second benefit is that, by restricting analyses to only human immunoglobulin sequences, the output will not be cluttered by the presence of rodent immunoglobulin sequences. There are far more rodent immunoglobulin sequences in databases than there are human.
(ii) List the human antibody variable domain sequences that have the most overall homology to the rodent antibody variable domain (from above). Do not make a distinction between homology within the framework regions and CDRs. Consider the overall homology.
(iii) Eliminate from consideration those human sequences that have CDRs that have a different length than those of the rodent CDRs. This rule does not apply to CDR 3, because the length of this CDR is normally quite variable. Also, there are sometimes no or very few human sequences that have the same CDR lengths as that of the rodent antibody. If this is the case, this rule can be loosened, and human sequences with one or more differences in CDR length can be allowed.
(iv) From the remaining human variable domains, one is selected that is most homologous to that of the rodent.
(v) The actual humanised antibody (the end result) should contain CDRs derived from the rodent antibody and a variable domain framework from the human antibody chosen above.
(vi) Instead of re-shaping or grafting to produce a humanised antibody, it would also be possible to synthesise the entire variable domain from scratch once the amino-acids of the non-human variable domain has been determined and the most homologous human variable domain has been identified.
(vii) If donor heavy chain has two small residues at positions 29 and 78, and recipient chain has large, typically aromatic, residues at one or both of these positions, then further analysis is required.
(viii) This analysis may take the form of a sequence comparison between the CDRH1 of the donor chain and that of other antibodies. For example, a CDRH1 sequence of SYGVH has been shown to require small residues at positions 29 and 78 for complete activity, and it is to be expected that other antibodies with the same or similar CDRH1 sequence will also require residues at these positions.

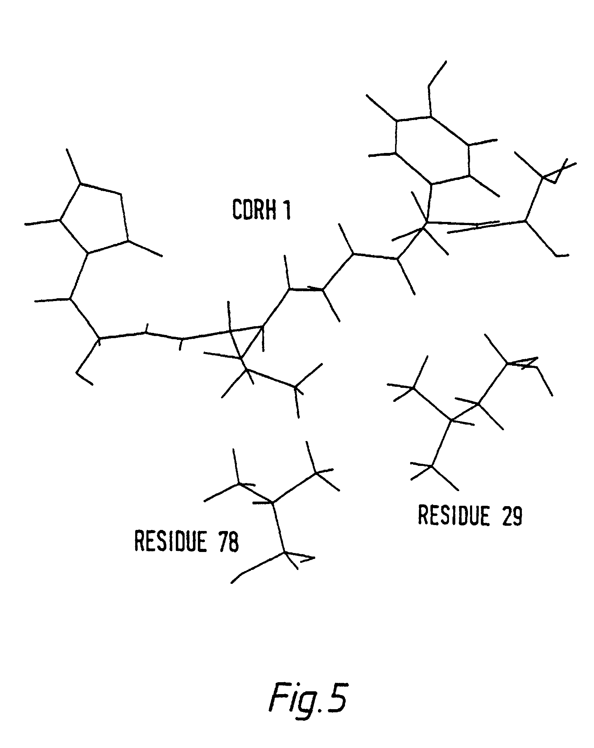

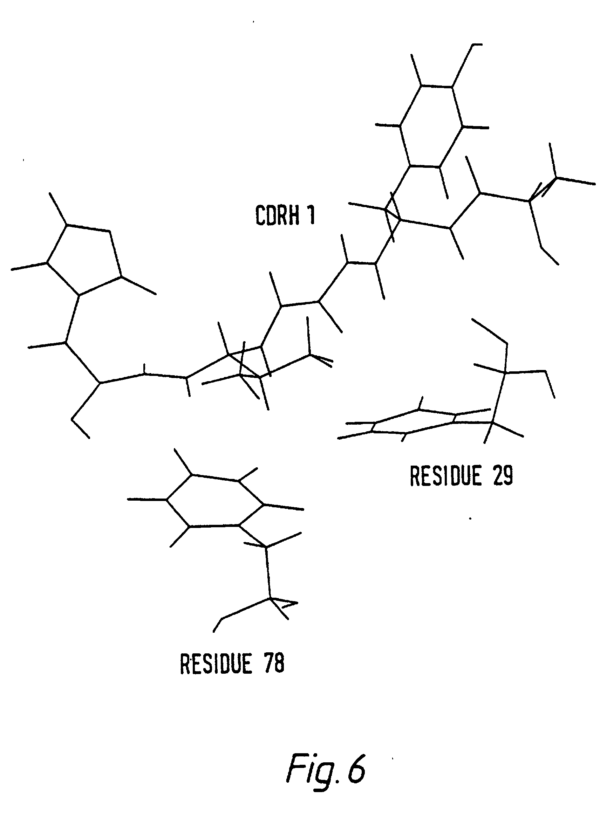

Alternatively, the analysis may take the form of detailed computer aided modelling of the CDRH1 region of the proposed humanised antibody using standard techniques (for example the *AbM* package from Oxford Molecular Ltd). If this analysis, for example, reveals that CDRH1 lies in close approximation to the packed side chains of residues 29 and 78, and that altering these residues from human to smaller residues changes the orientation or position of CDRH1, then such smaller residues should replace the human ones. An example of such a perturbation of CDRH1 is shown in Figures 5 and 6.

### Step 3: Grafting and re-shaping

See EP-A-0239400 and EP-A-054951 for details.

### Step 4: The transfection and expression of the altered antibody

Once the antibody has been humanised and residues 29 and/or 78 replaced, the cDNAs are linked to the appropriate DNA encoding light or heavy chain constant region, cloned into an expression vector, and transfected into mammalian cells. These steps can be carried out in routine fashion. A humanised antibody may therefore be prepared by a process comprising:
(a) preparing a first replicable expression vector including a suitable promoter operably linked to a DNA sequence which encodes at least a variable domain of an Ig heavy or light chain, the variable domain comprising framework regions from a human or primate antibody and CDRs comprising at least parts of the CDRs from a second antibody of different origin;
(b) if necessary, preparing a second replicable expression vector including a suitable promoter operably linked to a DNA sequence which encodes at least the variable domain of a complementary Ig light or heavy chain respectively;
(c) transforming a cell line with the first or both vectors; and
(d) culturing said transformed cell line to produce said altered antibody.

Preferably the DNA sequence in step (a) encodes both the variable domain and the or each constant domain of the antibody chain, the or each constant domain being derived from the human or primate antibody. The antibody can be recovered and purified. The cell line which is transformed to produce the altered antibody may be a Chinese Hamster Ovary (CHO) cell line or an immortalised mammalian cell line, which is advantageously of lymphoid origin, such as a myeloma, hybridoma, trioma, or quadroma cell line. The cell line may also comprise a normal lymphoid cell, such as a B-cell, which has been immortalised by transformation with a virus, such as the Epstein-Barr virus. Most preferably, the immortalised cell line is a myeloma cell line or a derivative thereof.

Although the cell line used to produce the altered antibody is preferably a mammalian cell line, any other suitable cell line, such as a bacterial cell line or a yeast cell line, may alternatively be used. In particular it is envisaged that E. coli-derived bacterial strains could be used.

Some immortalised lymphoid cell lines, such as myeloma cell lines, in their normal state secrete isolated Ig light or heavy chains. If such a cell line is transformed with the vector prepared in step (a), it may not be necessary to carry out step (b) of the process, provided that the normally secreted chain is complementary to the variable domain of the Ig chain encoded by the vector prepared in step (a). However, where the immortalised cell line does not secrete a complementary chain, it will be necessary to carry out (b). This step may be carried out by further manipulating the vector produced in step (a) so that this vector encodes not only the variable domain of an altered antibody light or heavy chain, but also the complementary variable domain.

Alternatively, step (b) is carried out by preparing a second vector which is used to transform the immortalised cell line. This alternative leads to easier construct preparation, but may be less preferred than the first alternative in that it may not lead to as efficient production of antibody.

Where the immortalised cell line secretes a complementary light or heavy chain, the transformed cell line may be produced for example by transforming a suitable bacterial cell with the vector and then fusing the bacterial cell with the immortalised cell line by spheroplast fusion. Alternatively, the DNA may be directly introduced into the immortalised cell line by electroporation or other suitable method.

The present process has been applied to obtain an antibody against the CD38 surface antigen.

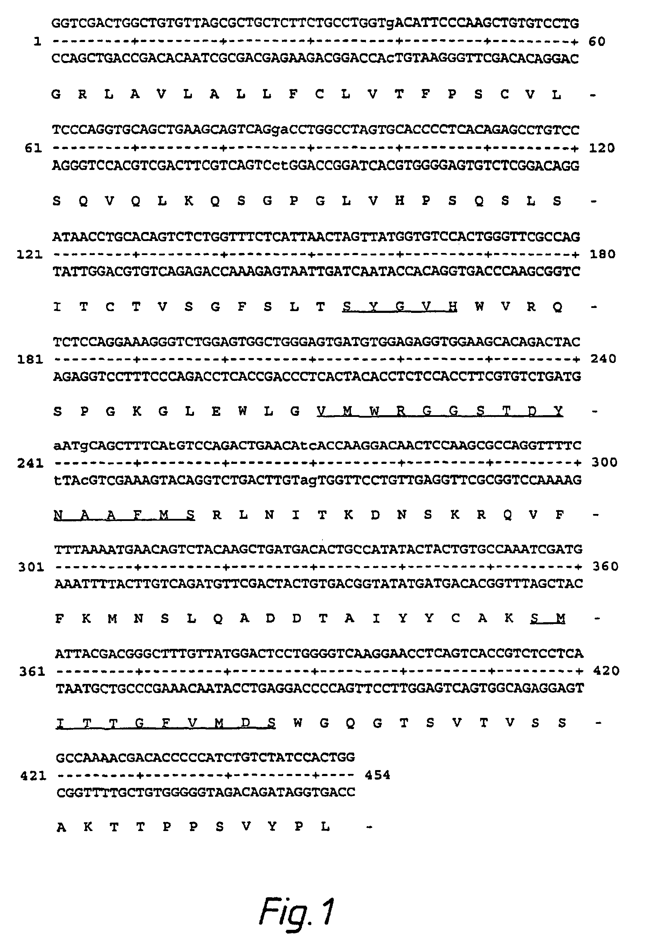

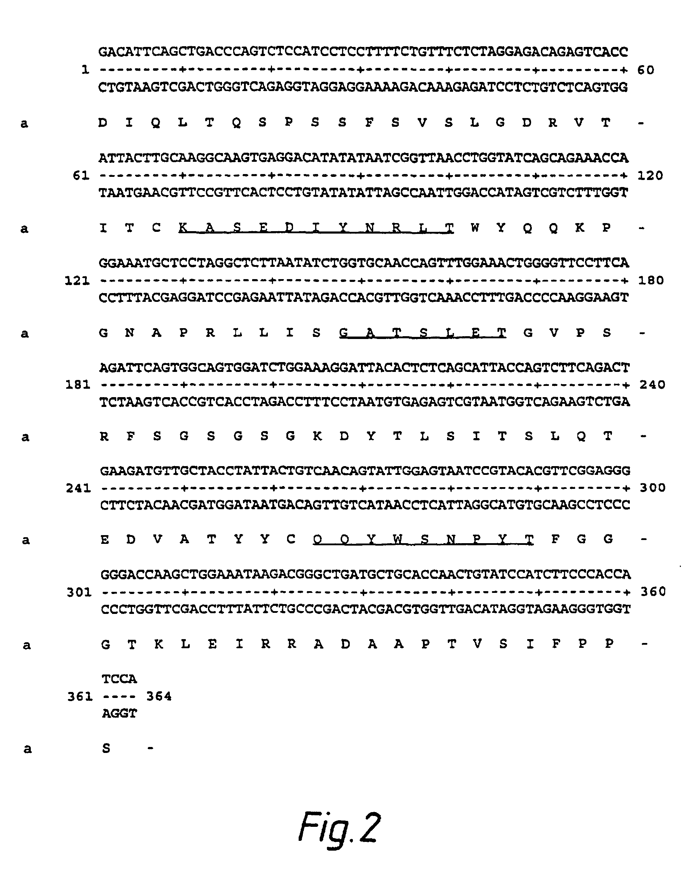

Briefly, a humanised anti-CD38 monoclonal antibody (termed h3S) was produced in the following fashion. cDNA was obtained from hybridoma cells secreting the murine monoclonal anti-(human CD38) AT13/5. cDNA clones encoding the heavy and light chains of the mouse antibody were identified and sequenced (Sequences 1 and 2 attached in Figures 1 and 2). This information was then used to choose appropriate human frameworks to receive the CDR grafts by the best-fit homology method. This procedure identified the REI light chain and the NEW heavy chain as the optimal choices.

CDRs were grafted on to the human frameworks. In addition, guided by published work (Riechman et al., 1988 Nature 332: 323 and Tempest et al., 1991, Bio/Technology 9:266), four framework changes were made at this stage at positions likely to affect antigen-binding: residues 27,30 and 94 in the heavy chain, and residue 49 in the light chain (numbering according to the Kabat system). The resulting humanised antibody was tested for CD38 binding, with negative results. Expression of the humanised light chain together with a chimeric heavy chain (murine VH, human CH) produced functional antibody, indicating that the humanisation of the light chain was adequate.

A further series of heavy chain framework changes were examined. In particular, the analysis identified a stretch of sequence from residue 66 to 73 which lies in close contact with CDRH2 and a pocket formed by the side chains of residues 29 and 78, lying close to CDRH1, as affecting antigen binding. As mentioned earlier on the importance of the regions 66-73 and 27-30 is recognised in the literature, though the role of residue 29 and 78 and the interaction between the side chains of residues 29 and 78 is not.

Although the invention is described with reference to an anti-CD38 antibody it is applicable to any antibody, whatever antigen it binds to. In particular any antibodies that bind the 40kD antigen (CO/17.1.A) as disclosed in J. Cell. Biol., 125 (2) 437-446, April 1994 and in Proc. Natl. Acad. Sci. 87, 3542-3546, May 1990, carcinoma antigens and antigens involved in autoimmune diseases. A specific example of an anti-40KD antibody is 323/A3.

Another example of an antibody is an anti-folate receptor antibody as disclosed in A. Tomasetti *et al*, Federation of European Biochemical Societies Vol 317, 143-146, Feb 1993. A specific example of an anti-folate antibody is MOV18. Further examples of antibodies include anti-CEA, anti mucin, anti-20/200KD, anti-ganglioside, anti-digoxin, anti-CD4 and anti-CD23.

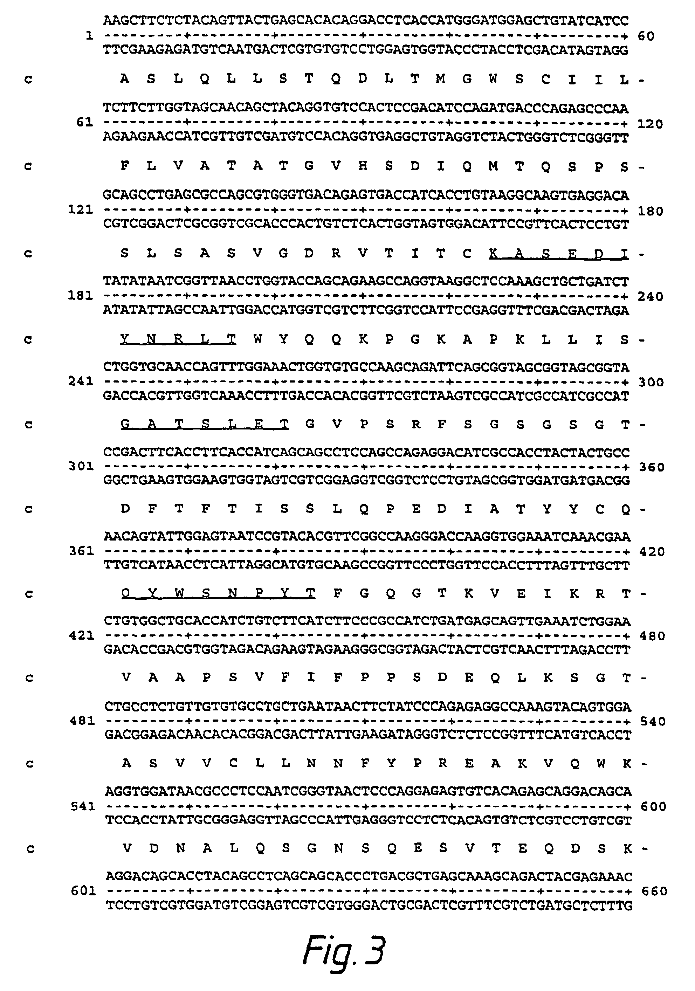

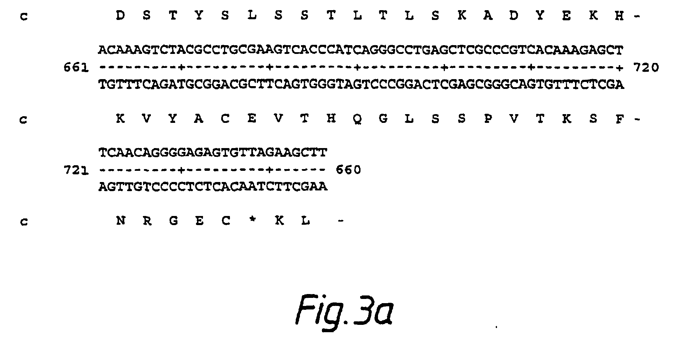

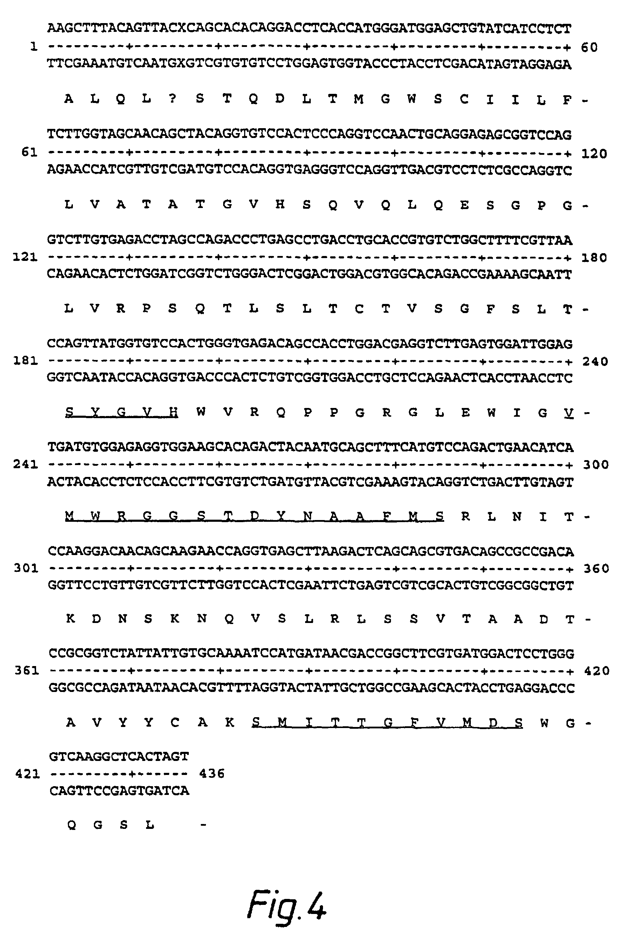

In particular the anti-CD38 antibody has the nucleotide sequences for the heavy chain and light chain variable region as shown in Figures 3, 3a and 4.

According to another aspect of the present invention there is provided the use of antibody according to the present invention in therapy. In particular there is provided the use of antibodies according to the invention for the treatment of cancer and their associated metastases and for treatment of autoimmune diseases, in particular for the treatment of multiple myeloma, lymphoma and rheumatoid arthritis.

The anti-CD38 antibody of the present invention can be used in the treatment of multiple myeloma.

CD38 is a transmembrane glycoprotein expressed by immature B lymphocytes, activated T and B lymphocytes, and plasma cells. Antibodies to CD38 capable of causing cell lysis may be useful in the immunotherapy of tumours bearing this antigen, principally multiple myeloma and 50% of non-Hodgin's lymphomas. Additionally, anti-CD38 antibodies may be useful in the treatment of autoimmune diseases such as rheumatoid arthritis and myaethenia gravis, as they have the potential to suppress both the humoral and cellular effector arms of the immune system.

A CD38 antibody according to the present invention has been demonstrated to be lytic for cells expressing CD38 on their surface. The humanised antibody has been shown to bind CD38 and compete with the parental antibody in CD38 binding.

Multiple myeloma is a neoplasm characterised by an accumulation of a clone of plasma cells, frequently accompanied by the secretion of immunoglobulin chains. Bone marrow invasion by the tumour is associated with anaemia, hypogammaglobinaemia and granulocytopaenia with concomitant bacterial infections. An abnormal cytokine environment, principally raised IL6 levels, often results in increased osteoclasis leading to bone pain, fractures and hypercalcaemia. Renal failure is not uncommon in the context of high concentrations of myeloma immunoglobulin and hypercalcaemia.

A variety of therapeutic protocols have been tried over recent years with little impact on the overall prognosis for myeloma patients. Treatment with melphalan and prednisolone remains the standard therapy, as it was thirty years ago (Bergsagel, 1989). A response to chemotherapy is associated with the induction of remission with median duration of about two years, but in all cases this is followed by eventual relapse and death (Alexanian and Dimopoulos, 1994 New England J. of Medicine Vol. 330 : 484). More aggressive chemotherapy utilising multiple cytotoxic agents has yielded little additional benefit in terms of survival or duration of remission, though high-dose therapy followed by autologous bone marrow transplant remains an area of active development.

Several workers have proposed immunotherapeutic strategies to combat myeloma. Interleukin 6 has been suggested to be a major growth factor for myeloma cells and may function in either an autocrine or paracrine fashion. Based on such results, interventions aimed at disrupting the IL6 signalling system have been designed. Two murine monoclonal that neutralise IL6 suppressed the proliferation of myeloma cells in a patient with leukaemic variant of the disease, though the tumour relapsed after 60 days.

Administration of anti-IL6 receptor monoclonal antibody to SCID mice engrafted with cells from a human myeloma cell line suppressed tumour growth, though only if the antibody was administered one day after injection of the myeloma cells. Antibody given after five days of growth had no significant effect. A CDR-grafted form of this antibody has also been prepared for possible human therapeutic use.

In a similar vein, myeloma cells bearing high levels of IL6 receptor have also been targeted by chimeric cytotoxinx consisting of IL6 variants linked to a modified form of Pseudomonas exotoxin. Cell killing is seen in vitro though the applicability of this technique in the clinic remains to be seen.

Our preference is for a more physiological approach, targeting myeloma cells for killing by the host immune system. The surface antigen CD38 is strongly expressed by more than 90% of multiple myeloma cells, and its suitability as a target for lytic immunotherapy has been discussed (Stevenson *et al*, 1991 Blood, Vol. 77, 5 : 1071-1079). The same report also demonstrated the competence of effector cells from myeloma patients for lysis of target cells coated with a chimeric anti-CD38.

The dosages of such antibodies will vary with the condition being treated and the recipient of the treatment, but will be in the range 1 to about 100 mg for an adult patient, preferably 1 - 10 mg, usually administered daily for a period between 1 and 30 days. A two part dosing regime may be preferable wherein 1 - 5 mg are administered for 5 - 10 days followed by 6 - 15 mg for a further 5 - 10 days.

Also included within the invention are formulation containing a purified preparation of an anti-CD38 antibody. Such formulation preferably include, in addition to antibody, a physiologically acceptable diluent or carrier possibly in admixture with other agents such as other antibodies or antibiotic. Suitable carriers include but are not limited to physiological saline, phosphate buffered saline, phosphate buffered saline glucose and buffered saline. Alternatively, the antibody may be lyophilised (freeze-dried) and reconstituted for use when needed, by the addition of an aqueous buffered solution as described above. Routes of administration are routinely parenteral including intravenous, intramuscular, subcutaneous and intraperitoneal injection or delivery.

The following Examples illustrate the invention. In the accompanying drawings:
Figure 1 shows the nucleotide and predicted amino acid sequence of mouse anti-CD38 antibody heavy chain variable region. The number of the first and last amino acid or nucleotide in each line is indicated in the left and right margins, respectively. CDRs are underlined.
Figure 2 shows the nucleotide and predicted amino acid sequence of mouse anti-CD38 antibody light chain variable region. The number of the first and last amino acid or nucleotide in each line is indicated in the left and right margins respectively. CDRs (underlined) were identified by comparison to known immunological sequences (Kabat *et al,* "Sequences of proteins of immunologic interest", US Dept of Health and Human Services, US Government Printing Office, 1987).
Figures 3 and 3a together show the nucleotide and predicted amino acid sequence of the humanised anti-CD38 antibody light chain cDNA. The number of the first and last amino acid or nucleotide in each line is indicated in the left and right margins, respectively. CDRs are underlined.
Figure 4 shows the nucleotide and predicted amino acid sequence of the humanised anti-CD38 antibody heavy chain cDNA. The number of the first and last amino acid or nucleotide in each line is indicated in the left and right margins, respectively. CDRs are underlined.
Figure 5 shows the configuration of the CDRHI (dark tubes) in the murine-anti-CD38 (murine residues at positions 29 and 78).
Figure 6 shows the configuration of the CDRHI (dark tubes) in the same region as Figure 5, but in a humanised construct with human residues at positions 29 and 78.
Figure 7 shows the effect of various heavy chain framework substitutions on relative binding affinity of anti-CD38 antibodies.

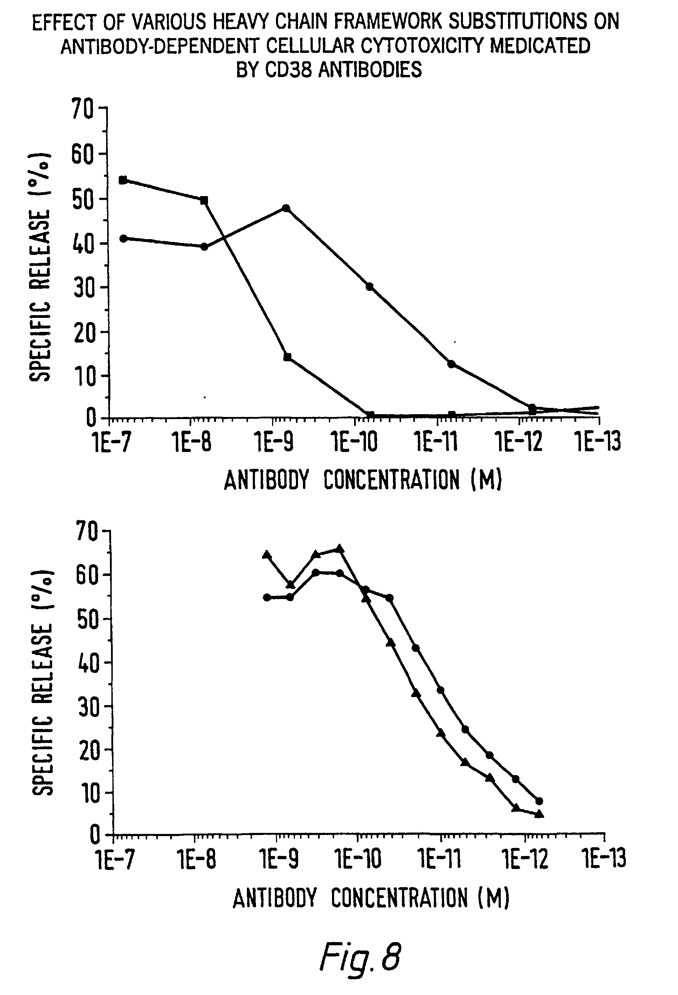

Figure 8 shows the effect of various heavy chain framework substitutions on antibody dependent cellular cytotoxicity mediated by CD38 antibodies.

### Examples

### Example 1

### Humanisation of anti-CD38 based on a mouse antibody (AT13/5:IgGLK)

### (a) General note on methodology

Unless otherwise stated, in the medodology described below, the following standard procedures and conditions were used. Manufacturers' recommended protocols were followed where applicable.

PCR experiments (Saiki *et al*, Science 239:487-491, 1988) were conducted using a programmable thermal cycler (Trio Biometra). A typical 100µl reaction mix contained 2.5 units of AmpliTag polymerase (Perkin-Elmer Cetus, Beaconsfield, UK) in the buffer supplied by the manufacturer; 250µM of each of dATP, dCTP, DGTP and dTTP, amplification primers at 1 µM, and template DNA. Unless otherwise noted, the following cycle specifications were used:
step 0: 94°C for 90 seconds
step 1: 94°C for 60 seconds
step 2: 50°C for 60 seconds, ramping up to step 3 at a rate of 0.15°C/second
step 3: 72°C for 60 seconds, go to step 1, repeating this loop for 25 cycles
step 4: 72°C for 10 minutes.

DNA sequencing was performed by the dideoxy method using the Sequenase v2 system (USB, Cambridge, UK), according to the manufacturer's instructions. The reaction products were separated on 8% acrylamide sequencing gels (Gel-Mix 8, BRL, Paisley, Scotland, UK).

To gel-purify DNA, one of two methods was used. For fragments smaller than 175 base-pairs, the DNA was separated on a conventional high-melting point agarose gel, and the DNA recovered using the Prep-a-Gene system (Bio-Rad Laboratories, Hemel Hempstead, UK). Larger fragments were purified by separation on a low-melting point agarose gel (NuSieve GTG, FMC, Rockland, ME), and the DNA recovered using Magic PCR Preps (Promega, Southampton, UK).

Numbering of amino-acid residues in antibody chains follows the scheme of Kabat et al ("Sequences of proteins of immunological interest", US Dept of Health and Human Services, US Government Printing Office, 1991).

### (b) Cloning and Sequencing of AT 13/5 antibody - Heavy Chain

Polyadenylated RNA was extracted from a culture containing 5 x 10⁶ of the AT13/5 mouse hybridoma line using a Micro Fast Tract kit (British Biotechnology, Oxford, UK). This was converted into oligo-dT-primed single-stranded cDNA using the SuperScript Preamplification system (BRL, Paisley, Scotland, UK). Aliquots of the resulting cDNA were used in PCRs designed to separately amplify the variable region of mouse immunoglobulin heavy and light chains.

The variable region of the heavy chain was amplified according to the method of Jones & Bendig (Bio/Technology 9:88-89), using a cocktail of primers specific to the signal peptide region (MHV1-12) and one primer specific for the mouse γl constant region (Mouse IgGl heavy chain reverse primer). The resulting PCR fragment was digested with Xma I and Sal I and cloned into pUC18. Clones obtained from two independent PCR reactions were sequenced on both strands and found to be identical implying that the sequence does not contain errors introduced by the PCR process. The complete sequence of the variable region appears as Figure 1.

### (c) Cloning and Sequencing of AT13/5 antibody - Light Chain

The sequence of the variable region of the light chain was also derived by a PCR-based cloning strategy using the same preparation of single-stranded cDNA as for the heavy chain. However, a more complex cloning and sequencing protocol was required, as the primers described by Jones & Bendig (*op cit*) preferentially amplify a non-productively rearranged kappa light chain from the AT13/5 cDNA. This chain arises from the fusion partner used to generate the AT13/5 hybridoma, here termed the MOPC-21 related V_{K}, and is of known sequence (Carroll, WL et. al., Molecular Immunology 25:991-995; 1988).

To amplify the cDNA encoding the anti-CD38 light chain a PCR was performed using the mouse kappa light chain reverse primer described by Jones & Bendig (*op cit*), and a primer VK1-BACK that hybridises to the framework 1 region of most mouse kappa chains (sequences: 5' GACATTCAGCTGACCCAGTCTCCA 3'). Conditions were as described for the heavy chain amplifications above, except that 35 cycles were used. These primers do not amplify the cDMA encoding the MOPC-21 related VK under these conditions.

An amplification fragment of the appropriate size was purified and a portion of this DNA used as the template for a second amplification (conditions as above, 30 cycles) using the light chain reverse primer and a variant of VK1-BACK containing a Hind III site (sequence: 5' GATCAAGCTTGACATTCAGCTGACCCAGTCTCCA 3'). The resulting fragment was digested with Hind III and Xma I and cloned into a pUC18. Clones were sequenced on both strands by the conventional dideoxy method. Additionally, a portion of the PCR product was directly sequenced using a thermal cycling strategy (fmol system, Promgea, Southampton, UK) with a primer (light chain reverse primer, as above) end-labelled with ³²P. The sequence obtained from the cycle sequencing experiment matched exactly the sequence derived by conventional methods.

Since this sequence was obtained from the products of two rounds of amplification, further confirmation of its accuracy was sought. The existing light chain sequence was used to design a primer that hydridises to the framework 1 region (sequence: 5' ACTAGTCGACCATCCTCCTTTTCTGTTTCTCTAGGAG 3'). This was used in conjunction with the light chain reverse primer in a PCR with the following cycle definition:
step O: 95°C for 120 seconds
step 1: 95°C for 60 seconds
step 2: 50°C for 60 seconds
step 3: 72°C for 60 seconds, go to step 1, repeating this loop for 30 cycles
step 4: 72°C for 10 minutes

Three independent reactions were performed, and after purification, the products were digested by Xma I and Sal I, and cloned into pUC18. Several clones were sequenced by the dideoxy method. All sequences so obtained were identical to those obtained previously, confirming that the proposed light chain sequence was indeed free from PCR errors. The complete sequence of the variable region of the light chain appears as Figure 2.

### (d) Design and construction of version 1 of the humanised antibody

Human variable domain frameworks were selected by the best-fit homology method (Lewis, AP & Crowe, JS in "Generation of Antibodies by Cell and Gene Immortalisation", Terhorst, C, Malavasi, F, Albertini, A (eds) Karger: Basel, 1993). The frameworks chosen for humanisation process were the light and heavy chain variable domains of Campath 1H (disclosed in EP-A-0328404). The humanised heavy and light chains were then constructed by a recombinant PCR technique (Lewis & Crowe, Gene 101:297-302, 1991).

### i) Light Chain

The primers used in the humanisation process were:

Primers A_{L} and H_{L} contain Hind III sites to allow cloning of the final amplficiation product. PCRs were performed according to the following cycle specification:
step 0: 95°C for 120 seconds
step 1: 95°C for 60 seconds
step 2: 45°C for 60 seconds
step 3: 72°C for 60 seconds, go to step 1, repeating this loop for 25 cycles
step 4: 72°C for 10 minutes

The template used in this reaction was DNA encoding the Campath 1H light chain, a construct in which the framework residues are taken from REI and the CDRs from a rat anti-human CDw52 antibody (Reichmann, L. *et. al*. Nature 332:323-337, 1988). The primers above are designed to wholly replace the Campath 1H sequence, leaving the AT13/5 CDRs grafted onto the REI frameworks.

Four initial PCRs were performed using long of template with the primer pairs: A_{L} and B_{L}, C_{L} and D_{L}, E_{L} and F_{L}, and G_{L} and H_{L}. The products of these reactions, AB_{L}, CD_{L}, EF_{L} and GH_{L} respectively were gel-purified and half of the amount recovered used in the second round of PCRs. Fragments AB_{L} and CD_{L} were used as template with primers A_{L} and D_{L} in one reaction, and fragments EF_{L} and GH_{L} were used as template with primers E_{L} and H_{L}. The reacton conditions were:
step 0: 95°C for 120 seconds
step 1: 95°C for 60 seconds
step 2: 45°C for 60 seconds
step 3: 72°C for 90 seconds, go to step 1, repeating this loop for 20 cycles

The products of these reactions, AD_{L} and EH_{L}, were gel-purified and half of each DNA used as template in a final reaction with primers A_{L} and H_{L} with the reaction conditions as for the second round PCR above. The resulting product was digested with Hind III and cloned into pUC18. A clone with the predicted structure as determined by complete sequence of the insert on both strands was chosen for further manipulation. The sequence of the variable region of this construct is given as Figures 3 and 3a.

### ii) Heavy Chain

The primers used in the humanisation process were:

PCRs were performed according to the following cycle specification:
step 0: 95°C for 120 seconds
step 1: 95°C for 60 seconds
step 2: 45°C for 60 seconds
step 3: 72°C for 60 seconds, go to step 1, repeating this loop for 25 cycles
step 4: 72°C for 10 minutes

The template used in this reaction was DNA encoding the Campath 1H heavy chain, a construct in which the CDRs and framework residues 27 and 30 are taken from a rat anti-human CDw52 antibody (Reichmann, L *et. al.* op cit), and the remainder of the framework residues from NEW. The primers above are designed to replace the Campath 1H CDR sequences, leaving the AT13/5 CDRs grafted onto the Campath 1H framework. Also, heavy chain residue 94 is known to be important in antigen-binding (Tempest, PR *et. al*., Bio/Technology, 9:260-271, 1991), so the AT13/5 sequence was adopted at this position. The rat sequence at residues 27 and 30 is more homologous to the AT13/5 sequence than is the unmodified NEW sequence. Primers A_{H} and H_{H} contains Hind III and EcoR I sites respectively. Additionally, primer G_{H} engineers a Spel site into the framework 4 region to allow coupling to a previously prepared human C_{H} sequence.

Four initial PCRs were performed using long of template with the primer pairs: A_{H} and B_{H}, C_{H} and D_{H}, E_{H} and F_{H}, and G_{H} and H_{H}. The products of these reactions, AB_{H}, CD_{H} were used as template with primers A_{H} and D_{H} in one reaction, and fragments EF_{H} and GH_{H} were used as template with primers E_{H} and H_{H}. The reaction conditions were:
step 0: 95°C for 120 seconds
step 1: 95°C for 60 seconds
step 2: 45°C for 60 seconds
step 3: 72°C for 90 seconds, go to step 1, repeating this loop for 20 cycles

The products of these reactions, AD_{H} and EH_{H}, were gel-purified and half of each DNA used as template in a final reaction with primers A_{H} and H_{H} with the reaction conditions as for the second round PCR above. The resulting product was digested with Hind III and Spe I, and the fragment containing the variable region cloned into a pUC18-based vector containing the human C_{H} sequence. A clone with the predicted structure as determined by complete sequencing of the insert on both strands was chosen for further manipulation.

### (e) Eukaryotic expression of version 1 of the humanised antibody

Humanised AT13/5 heavy and light chains were cloned into eukaryotic expression vectors under human β actin promoters. The heavy and light chain plasmids were transiently expressed in B11 CHO cells by cotransfection of the two plasmids using Transfectam (Promega, Southampton, UK). Culture supernatants were assayed for human IgG by ELISA, and tested for CD38-binding activity by FACS analysis using the CD38-positive B-cell line Wien 133.

Although the culture supernatants contained significant amounts of human IgG, no anti-CD38 activity could be detected by FACS, even when supernatants were concentrated 10-fold. This result suggests that simple grafting of the CDRs from AT13/5 onto the Campath 1H and REI human frameworks is insufficient to transfer the antibody specificity. A series of framework changes were therefore undertaken in order to restore CD38-binding activity.

### (f) Framework changes

Since most of the framework residues previously shown to be important in restoring antigen binding are in the heavy chain variable region, it was decided to focus on this part of the antibody. Additional cotransfection of the humanised light chain with a chimaeric heavy chain construct (mouse heavy variable region fused to human C_{H}), produced active antibody (hereafter termed hybrid antibody) that bound CD38 with an affinity comparable to that of the original mouse antibody. The region with the lowest homology between the human frameworks used and the original mouse sequence is also close to some residues of known importance. This region, just downstream of the CDR3 sequence was chosen for mutagenesis.

Heavy chain residues 67 to 71 inclusive and 73 were grafted from the mouse antibody onto the humanised heavy chain using recombinant PCR. The primers used were as follows:

Two initial PCRs were performed using 10ng of version 1 humanised heavy chain template with the primer pairs: A_{H} and I_{H} and J_{H} and H_{H}. The products of these reactions, AI_{H} and JH_{H} respectively, were gel-purified and half of the recovered DNA used in a second round of PCR with primers A_{H} and H_{H} to generate version 2 of the humanised heavy chain variable region. This was cloned, sequenced, transferred to the expression system, and then transiently co-expressed with the humanised light chain construct as above. Once again, culture supernatant from transfected CHO cells produced human IgG as determined by ELISA, but no CD38-binding activity could be detected by FACS analysis.

A further round of mutations based on both version 1 and version 2 of the humanised heavy chain were then produced by a method identical to that described above. A total of six version 3 heavy chains were produced in which the following heavy chain framework residues were grafted from the mouse sequence onto one or other humanised sequence:

| Antibody | Template for mutagenesis | Grafted residues | Primers used |
|---|---|---|---|
| h3J | version 1 | 28,29 | K_{H},L_{H} |
| h3K | version 2 | 28,29 | K_{H},L_{H} |
| h3L | version 1 | 76 | M_{H},O_{H} |
| h3M | version 2 | 76 | N_{H},O_{H} |
| h3N | version 1 | 28,29,76 | K_{H},L_{H},M_{H},O_{H} |
| h3O | version 2 | 28,29,76 | K_{H},L_{H},N_{H},O_{H} |

Additionally, all constructions used primers A_{H} and H_{H}. The primer sequences used were:

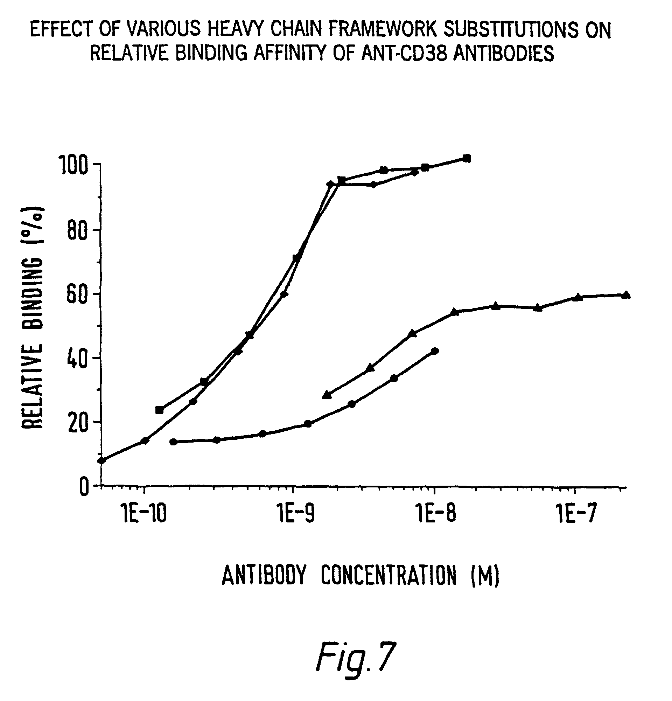

Heavy chain constructs containing the expected sequence were transferred into mammalian expression vectors, and cotransfected with the humanised light chain construct into CHO cells, as above. Tissue culture supernatants containing human IgG as determined by ELISA were assayed for CD38-binding activity by FACS. Constructs h3K and h30 showed antigen-binding in this assay though with less activity than the hybrid antibody (see Fig. 7).

### (g) Method for changing framework residues at positions 29 and 78

In order to establish why h30 showed less activity than the hybrid antibody further sequences analysis suggested potential problems with positions 29 and 78 in the heavy chain.

Having identified mutations to be made in the heavy chain framework regions, these can be produced by a variety of standard methods: examples being site-directed mutagenesis, recombinant PCR and gene synthesis using oligonucleotides. In the case of the anti-CD38 heavy chain VH, recombinant PCR was used to introduce murine residues at positions 28-29 and 78 sequentially.

A human anti-CD38 heavy chain VH already incorporating murine residues at positions 27, 30, 67, 68, 69, 70, 71, 73 and 94 (Version 2 as described in (f) above) was used as template for the first round of mutagenesis. This was amplified with the following PCR primers in two separate reactions:

In primers B and C, the triplets encoding the murine residues at positions 28 and 29 are underlined. In the first reaction, the template was amplified with primers A and B. In the second reaction, the template was amplfified with primers C and D. The products of the two reactions were purified, mixed, and amplified with primers A and D. The reaction product was purified, cleaved with Hind III and SpeI, and the 450 base-pair fragment encoding the VH cloned into a variant of pUC18 containing a human γl cDNA cassette (Sime *et al*, 1993; J. Immunol, 151:2296). Clones were sequenced to ensure correct introduction of the murine residues at positions 28 and 29.

A clone incorporating these changes was then used as template for a second round of recombinant PCR mutagenesis to introduce the murine residue at position 78. A procedure identical to that described above was followed, except that primers B and C were replaced by primers E and F respectively, which contain a triplet (underlined) that incorporates the murine residue at position 78.

The resulting heavy chain (see Fig. 4) when co-expressed with the humanised light chain (see Fig. 3) produces humanised anti-CD38,h3S.

### (h) Eukaryotic expression of functional humanised antibody

To creat clonal cell lines for further characterisation, plasmids encoding the humanised h3S heavy chain and the chimaeric heavy chain were separately co-transfected with the humanised light chain into B11 CHO cells.

### Example 2

### Biological activity

### (a) CD38 Binding Studies

(i) Effect of various heavy chain framework substitutions on relative binding affinity of anti-CD38 antibodies.

Binding was assessed by FACS staining of CD38 positive cells.

Heavy chains incorporating one or more of mouse framework residues were created as described above and combined with the humanised light chain to make antibodies which were assayed for binding to CD38, with the following results.

| Construct | 66-73 | 28/29 | 78 | Binding |
|---|---|---|---|---|
| h1 | - | - | - | - |
| h2 | + | - | - | - |
| h3J | - | + | - | - |
| h3K | + | + | - | + |
| h3S | + | + | + | ++ |

In this table, + denotes that the murine framework residue is incorporated into the humanised antibody at the indicated position, - denotes that the human residue remains.

### Discussion

According to computer modelling studies the change of the 66-73 region back to mouse framework causes the humanised CDRH2 to adopt a similar conformation to that of the mouse antibody. However, as the construct h2 shows, this is insufficient to obtain binding. The model also suggests that in the mouse anti-CD38 antibody, positions 29 and 78 are occupied by small residues, whose side-chains pack neatly together allowing CDRH1 to adopt the correct configuration for antigen binding. In the humanised constructs h1 and h2, the side chains are unable to pack together in this fashion, being much larger, and so distort CDRH1, preventing antigen binding. This aspect of the model is illustrated in Figures 5 and 6 (attached). Figure 5 shows the configuration of CDRH1 (dark tubes) in the murine anti-CD38. In Figure 6 showing the same region in a humanised construct with human residues at positions 29 and 78, the extra bulk of these side chains has clearly resulted in a distortion of the CDRH1 conformation.

Partial relief of this effect can be obtained by using the murine residue at position 29 and the human residue at position 78, though the resulting antibody shows markedly reduced function. Use of murine residues at both positions 29 and 78 restores activity, as evidenced by the data for the h3S construct.

(ii) Anti-CD38 heavy chain variable regions were fused to human γl constant region and coexpressed in CHO cells with humanised anti-CD38 light chain. CD38-binding activity is expressed normalised to the signal obtained using a saturating dose of hybrid antibody (mouse VH) in the same experiment.

Results are shown in Figure 7 where:
◆ Humanised antibody with murine residues at 28,29 and 78
▲ Humanised antibody with murine residues at 28,29 and 76
● Humanised antibody with murine residues at 28,29
■ Hybrid antibody

In addition to the above substitutions, all humanised heavy chains contained murine framework residues at positions 27, 30, 67, 68, 69, 70, 71, 73 and 94. These alone are insufficient to obtain detectable binding by FACS.

These results demonstrate the critical importance of the small residues at positions 29 and/or 78 in obtaining full humanised heavy chain activity. They also demonstrate the specific nature of the interaction, in that a murine residue at position 76 close to position 78 was unable to restor activity.

(b) Effect of various heavy chain framework substitutions on antibody-dependent cellular cytotoxicity mediated by CD38 antibodies.

Antibody-dependent cellular cytotoxicity is normally assessed by one of several label-release techniques, well-known in the literature. In one such assay, 10⁴ target cells (Wien 133) were labelled with europium and then exposed to freshly prepared human peripheral blood lymphocytes in the presence of antibody as an effector:target ratio of 50:1. Lysis was estimated by detecting release of europium after 4 hours, and quantitated by reference to control reactions without antibody or peripheral blood lymphocytes or with detergent such as Triton-X100.

The effect of framework substitutions on the lytic potential of humanised anti-CD38 monoclonals was examined in label-release assay. Wien 133 target cells were loaded with label (either 51Cr or Eu) and then exposed to freshly prepared human peripheral blood mononuclear cells in the presence of varying amounts of anti-CD38 antibody. Cytotoxicity is expressed as the proportion of total releasable label liberated by antibody treatment.

Results are shown in Figure 10 where:
▲ Humanised antibody with murine residues at 28,29 and 78
■ Humanised antibody with murine residues at 28,29 and 76
● Hybrid antibody

These results show that the combination of framework changes at positions 29 and 78 confer full activity on the humanised heavy chain for cytotoxic function. Although incorporation of a small murine residue at position 29 results in some binding activity (Figure 7), this is insufficient to achieve full effector function.

## Claims

1. A monoclonal antibody having donor CDRS of foreign origin and a recipient framework having a sequence of human or primate origin wherein an original amino acid residue of the recipient framework region of the heavy chain is replaced by a replacement amino acid residue that is the same or similar to that in the corresponding position of the sequence of the corresponding framework region of the heavy chain from which the CDRs are derived **characterised in that** the replaced original amino acid residue consists of positions 29 and 78, the numbering according to the Kabat system.

2. A monoclonal antibody according to claim 1 wherein one or both of the original amino acid residues of the recipient framework region are replaced by replacement amino acid residues of similar size hydrophobicity and charge to the amino acids in the corresponding positions of the corresponding framework region of the antibody from which the CDRs are derived.

3. A monoclonal antibody according to any of the preceding claims, wherein the original amino acid residues of the recipient framework region are the same or different and are tyrosine, histidine, tryptophan or 2-phenyl-alanine.

4. A monoclonal antibody according to claim 3, wherein the replacement amino acid residues are the same or different and are selected from glycine, alanine, valine, serine or leucine.

5. A monoclonal antibody according to any of the preceding claims wherein the recipient framework region is from a heavy chain selected from LES-C, T52, Ab44, HIGI and NEW.

6. A monoclonal antibody according to any of the preceding claims, wherein the CDRs are of rat, mouse rabbit, or hamster origin.

7. A monoclonal antibody according to any of the preceding claims, wherein the heavy chain of the antibody from which the CDRs are derived is a murine heavy chain in Kabat groups IB and IIC.

8. A monoclonal antibody according to any of the preceding claims wherein the antibody binds to CD38.

9. A monoclonal antibody according to claim 8 having a nucleotide sequence as shown in figures 3, 3a and 4.

10. A monoclonal antibody according to any of the preceding claims, wherein the donor CDR is CDRHI.

11. A monoclonal antibody according to claim 10, wherein CDRHI has a sequence of SYGVH.

12. A method of restoring immunoactivity in an antibody comprising donor CDRs of foreign origin and a recipient framework region having a sequence of human or primate origin comprises the steps of:
(i) obtaining the sequence of a donor heavy chain;
(ii) selecting a recipient human or primate framework, by best-fit homology method;
(iii) replacing an amino acid residue in position 29 and 78 only of the sequence of the recipient framework region of the heavy chain by an amino acid that is the same or similar to that in the corresponding position of the sequence of the corresponding framework region of the antibody from which the CDRs are derived;
(iv) grafting donor CDRs into the recipient human framework.

13. Use of an antibody according to any of the preceding claims in the manufacture of a medicament for the treatment of cancer and autoimmune diseases.

14. Use of an antibody according to claim 8 or 9 in the manufacture of a medicament for treatment of multiple myeloma, lymphoma and autoimmune diseases.

15. Use of an antibody according to any of claims 1 to 11 for the manufacture of a medicament for the treatment of multiple myeloma, lymphoma, or rheumatoid arthritis.

16. A pharmaceutical composition comprising an antibody according to any of claims 1 to 11 and a physiologically acceptable diluent or carrier.

## Patentansprüche

1. Monoklonaler Antikörper mit Donor-CDRS fremden Ursprungs und einem Empfänger-Gerüst mit einer Sequenz menschlichen oder Primaten-Ursprungs, worin ein ursprünglicher Aminosäure-Rest der Empfänger-Gerüstregion der schweren Ketten gegen einen Austausch-Aminosäure-Rest ausgetauscht ist, der der gleiche oder ähnlich demjenigen in der entsprechenden Position der Sequenz der entsprechenden Gerüstregion der schweren Kette ist, aus der die CDRs stammen, **dadurch gekennzeichnet, daß** der ausgetauschte ursprüngliche Aminosäure-Rest aus den Positionen 29 und 78 besteht, mit der Numerierung gemäß dem Kabat-System.

2. Monoklonaler Antikörper gemäß Anspruch 1, worin einer oder beide der ursprünglichen Aminosäure-Reste der Empfänger-Gerüstregion gegen Austausch-Aminosäure-Reste ähnlicher Größe, Hydrophobie und Ladung wie die Aminosäuren in den entsprechenden Positionen der entsprechenden Gerüstregion des Antikörpers ausgetauscht sind, aus dem die CDRs stammen.

3. Monoklonaler Antikörper gemäß einem der vorhergehenden Ansprüche, worin die ursprünglichen Aminosäure-Reste der Empfänger-Gerüstregion die gleichen oder verschieden sind und Tyrosin, Histidin, Tryptophan oder 2-Phenylalanin sind.

4. Monoklonaler Antikörper gemäß Anspruch 3, worin die Austausch-Aminosäure-Reste die gleichen oder verschieden sind und aus Glycin, Alanin, Valin, Serin oder Leucin ausgewählt sind.

5. Monoklonaler Antikörper gemäß einem der vorhergehenden Ansprüche, worin die Empfänger-Gerüstregion aus einer schweren Kette ist, ausgewählt aus LES-C, T52, Ab44, HIGI und NEW.

6. Monoklonaler Antikörper gemäß einem der vorhergehenden Ansprüche, worin die CDRs aus Ratte, Maus, Kaninchen oder Hamster stammen.

7. Monoklonaler Antikörper gemäß einem der vorhergehenden Ansprüche, worin die schwere Kette des Antikörpers, aus dem die CDRs stammen, eine schwere Kette der Maus in Kabat-Gruppen IB und IIC ist.

8. Monoklonaler Antikörper gemäß einem der vorhergehenden Ansprüche, worin der Antikörper an CD38 bindet.

9. Monoklonaler Antikörper gemäß Anspruch 8 mit einer Nukleotidsequenz wie in den Figuren 3, 3a und 4 gezeigt.

10. Monoklonaler Antikörper gemäß einem der vorhergehenden Ansprüche, worin die Donor-CDR CDRHI ist.

11. Monoklonaler Antikörper gemäß Anspruch 10, worin CDRHI eine Sequenz SYGVH hat.

12. Verfahren zur Wiederherstellung der Immunoaktivität in einem Antikörper, umfassend Donor-CDRs fremden Ursprungs und eine Empfänger-Gerüstregion mit einer Sequenz menschlichen oder Primaten-Ursprungs, umfassend die folgenden Schritte:
(i) Erhalten der Sequenz aus einer schweren Donor-Kette;
(ii) Auswählen eines menschlichen oder Primaten-Empfängergerüsts durch das Verfahren der "Best-Fit"-Homologie;
(iii) Austauschen eines Aminosäure-Restes in der Position 29 und 78 nur aus der Sequenz der Empfänger-Gerüstregion der schweren Kette gegen eine Aminosäure, die gleich oder ähnlich derjenigen in der entsprechenden Position der Sequenz der entsprechenden Gerüstregion des Antikörpers ist, aus dem die CDRs stammen;
(iv) Verpflanzen von Donor-CDRs in das menschliche Empfänger-Gerüst.

13. Verwendung eines Antikörpers gemäß einem der vorhergehenden Ansprüche in der Herstellung eines Medikaments zur Behandlung von Krebs und Autoimmunerkrankungen.

14. Verwendung eines Antikörpers gemäß Anspruch 8 oder 9 in der Herstellung eines Medikaments zur Behandlung von multiplem Myelom, Lymphom und Autoimmunerkrankungen.

15. Verwendung eines Antikörpers gemäß einem der Ansprüche 1 bis 11 zur Herstellung eines Medikaments zur Behandlung von multiplem Myelom, Lymphom oder rheumatoider Arthritis.

16. Pharmazeutische Zusammensetzung umfassend einen Antikörper gemäß einem der Ansprüche 1 bis 11 und einen physiologisch akzeptablen Verdünnungsstoff oder Träger.

## Revendications

1. Anticorps monoclonal ayant des CDR de donneur d'origine étrangère et une charpente réceptrice ayant une séquence d'origine humaine ou primate, dans lequel un résidu amino-acide original de la région charpente réceptrice de la chaîne lourde est remplacé par un résidu amino-acide de remplacement qui est identique ou qui est semblable à celui de la position correspondante de la séquence de la région charpente correspondante de la chaîne lourde d'où sont dérivées les CDR, **caractérisé en ce que** le résidu amino-acide original remplacé concerne les positions 29 et 78, selon la numérotation du système Kabat.

2. Anticorps monoclonal selon la revendication 1, dans lequel un ou les deux résidus amino-acide originaux de la région charpente réceptrice sont remplacés par des résidus amino-acide de remplacement, de taille, d'hydrophobie et de charge similaires à celles des amino-acides des positions correspondantes de la région charpente correspondante de l'anticorps dont sont dérivées les CDR.

3. Anticorps monoclonal selon l'une quelconque des revendications précédentes, dans lequel les résidus amino-acide originaux de la région charpente réceptrice sont identiques ou différents, et sont la tyrosine, l'histidine, le tryptophane ou la 2-phényl-alanine.

4. Anticorps monoclonal selon la revendication 3, dans lequel les résidus amino-acide de remplacement sont identiques ou différents, et sont choisis parmi la glycine, l'alanine, la valine, la sérine ou la leucine.

5. Anticorps monoclonal selon l'une quelconque des revendications précédentes, dans lequel la région charpente réceptrice provient d'une chaîne lourde choisie parmi LES-C, T52, Ab44, HIGI et NEW.

6. Anticorps monoclonal selon l'une quelconque des revendications précédentes, dans lequel les CDR sont originaires du rat, de la souris, du lapin ou du hamster.

7. Anticorps monoclonal selon l'une quelconque des revendications précédentes, dans lequel la chaîne lourde de l'anticorps dont sont dérivées les CDR, est une chaîne lourde murine des groupes Kabat IB et IIC.

8. Anticorps monoclonal selon l'une quelconque des revendications précédentes, dans lequel l'anticorps se lie au CD38.

9. Anticorps monoclonal selon la revendication 8, ayant une séquence nucléotide telle que représentée dans les Figures 3, 3a et 4.

10. Anticorps monoclonal selon l'une quelconque des revendications précédentes, dans lequel le CDR du donneur est la CDRHI.

11. Anticorps monoclonal selon la revendication 10, dans lequel la CDRHI a une séquence SYGVH.

12. Technique de restauration de l'immuno-activité dans un anticorps comprenant des CDR de donneurs d'origine étrangère et une région charpente réceptrice ayant une séquence d'origine humaine ou primate comprenant les étapes :
(i) d'obtention de la séquence d'une chaîne lourde de donneur ;
(ii) de sélection d'une charpente réceptrice humaine ou primate par la technique d'homologie optimale ;
(iii) de remplacement d'un résidu amino-acide en position 29 et en position 78 uniquement, de la séquence de la région charpente réceptrice de la chaîne lourde, par un amino-acide qui est identique ou semblable à celui de la position correspondante de la séquence de la région charpente correspondante de l'anticorps dont sont dérivées les CDR;
(iv) de greffage des CDR du donneur dans la charpente humaine réceptrice.

13. Utilisation d'un anticorps selon l'une quelconque des revendications précédentes, dans la fabrication d'un médicament pour le traitement du cancer et des maladies auto-immunes.

14. Utilisation d'un anticorps selon la revendication 8 ou la revendication 9, dans la fabrication d'un médicament pour le traitement du myélome multiple, du lymphome et des maladies auto-immunes.

15. Utilisation d'un anticorps selon l'une quelconque des revendications 1 à 11, pour la fabrication d'un médicament pour le traitement du myélome multiple, du lymphome et de l'arthrite rhumatoïde.

16. Composition pharmaceutique comprenant un anticorps selon l'une quelconque des revendications 1 à 11, et un diluant ou véhicule physiologiquement acceptable.


### SEQUENCE LISTING

(1) GENERAL INFORMATION:
   (i) APPLICANT:
      (A) NAME: The Wellcome Foundation Limited
      (B) STREET: Unicorn House, PO Box 129, 160 Euston Road
      (C) CITY: London
      (D) STATE: not applicable
      (E) COUNTRY: United Kingdom
      (F) POSTAL CODE (ZIP): NW1 2BP

      (A) NAME: ELLIS, Jonathan Henry
      (B) STREET: The Wellcome Research Laboratories, Langley Court
      (C) CITY: South Eden Park Road, Beckenham
      (D) STATE: Kent
      (E) COUNTRY: United Kingdom
      (F) POSTAL CODE (ZIP): BR3 3BS

      (A) NAME: LEWIS, Alan Peter
      (B) STREET: The Wellcome Research Laboratories, Langley Court
      (C) CITY: South Eden Park Road,Beckenham
      (D) STATE: Kent
      (E) COUNTRY: United Kingdom
      (F) POSTAL CODE (ZIP): BR3 3BS
   (ii) TITLE OF INVENTION: ANTIBODIES
   (iii) NUMBER OF SEQUENCES: 46
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 454 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION:1..453
   (xi) SEQUENCE DESCRIPTION: SEQ ID NO: 1:
   GGT CGA CTG GCT GTG TTA GCG CTG CTC TTC TGC CTG GTG ACA TTC CCA      48
   Gly Arg Leu Ala Val Leu Ala Leu Leu Phe Cys Leu Val Thr Phe Pro
     1               5                  10                  15
   AGC TGT GTC CTG TCC CAG GTG CAG CTG AAG CAG TCA GGA CCT GGC CTA      96
   Ser Cys Val Leu Ser Gln Val Gln Leu Lys Gln Ser Gly Pro Gly Leu
                20                  25                  30
   GTG CAC CCC TCA CAG AGC CTG TCC ATA ACC TGC ACA GTC TCT GGT TTC     144
   Val His Pro Ser Gln Ser Leu Ser Ile Thr Cys Thr Val Ser Gly Phe
            35                  40                  45
   TCA TTA ACT AGT TAT GGT GTC CAC TGG GTT CGC CAG TCT CCA GGA AAG     192
   Ser Leu Thr Ser Tyr Gly Val His Trp Val Arg Gln Ser Pro Gly Lys
   50 55 60
   GGT CTG GAG TGG CTG GGA GTG ATG TGG AGA GGT GGA AGC ACA GAC TAC 240
   Gly Leu Glu Trp Leu Gly Val Met Trp Arg Gly Gly Ser Thr Asp Tyr
   65 70 75 80
   AAT GCA GCT TTC ATG TCC AGA CTG AAC ATC ACC AAG GAC AAC TCC AAG 288
   Asn Ala Ala Phe Met Ser Arg Leu Asn Ile Thr Lys Asp Asn Ser Lys
   85 90 95
   CGC CAG GTT TTC TTT AAA ATG AAC AGT CTA CAA GCT GAT GAC ACT GCC 336
   Arg Gln Val Phe Phe Lys Met Asn Ser Leu Gln Ala Asp Asp Thr Ala
   100 105 110
   ATA TAC TAC TGT GCC AAA TCG ATG ATT ACG ACG GGC TTT GTT ATG GAC 384
   Ile Tyr Tyr Cys Ala Lys Ser Met Ile Thr Thr Gly Phe Val Met Asp
   115 120 125
   TCC TGG GGT CAA GGA ACC TCA GTC ACC GTC TCC TCA GCC AAA ACG ACA 432
   Ser Trp Gly Gln Gly Thr Ser Val Thr Val Ser Ser Ala Lys Thr Thr
   130 135 140
   CCC CCA TCT GTC TAT CCA CTG G 454
   Pro Pro Ser Val Tyr Pro Leu
   145 150
(2) INFORMATION FOR SEQ ID NO: 2:
   (i) SEQUENCE CHARACTERISTICS:
      (A) LENGTH: 151 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Gly Arg Leu Ala Val Leu Ala Leu Leu Phe Cys Leu Val Thr Phe Pro
   1 5 10 15
   Ser Cys Val Leu Ser Gln Val Gln Leu Lys Gln Ser Gly Pro Gly Leu
   20 25 30
   Val His Pro Ser Gln Ser Leu Ser Ile Thr Cys Thr Val Ser Gly Phe
   35 40 45
   Ser Leu Thr Ser Tyr Gly Val His Trp Val Arg Gln Ser Pro Gly Lys
   50 55 60
   Gly Leu Glu Trp Leu Gly Val Met Trp Arg Gly Gly Ser Thr Asp Tyr
   65 70 75 80
   Asn Ala Ala Phe Met Ser Arg Leu Asn Ile Thr Lys Asp Asn Ser Lys
   85 90 95
   Arg Gln Val Phe Phe Lys Met Asn Ser Leu Gln Ala Asp Asp Thr Ala
   100 105 110
   Ile Tyr Tyr Cys Ala Lys Ser Met Ile Thr Thr Gly Phe Val Met Asp
   115 120 125
   Ser Trp Gly Gln Gly Thr Ser Val Thr Val Ser Ser Ala Lys Thr Thr
   130 135 140
   Pro Pro Ser Val Tyr Pro Leu
   145 150
(2) INFORMATION FOR SEQ ID NO: 3:
   (i) SEQUENCE CHARACTERISTICS:
      (A) LENGTH: 454 base pairs
      (B) TYPE: nucleic acid
      (C) STRANDEDNESS: double
      (D) TOPOLOGY: linear
   (iv) ANTI-SENSE: YES
   (xi) SEQUENCE DESCRIPTION: SEQ ID NO: 3:
   CCAGCTGACC GACACAATCG CGACGAGAAG ACGGACCACT GTAAGGGTTC GACACAGGAC      60
   AGGGTCCACG TCGACTTCGT CAGTCCTGGA CCGGATCACG TGGGGAGTGT CTCGGACAGG     120
   TATTGGACGT GTCAGAGACC AAAGAGTAAT TGATCAATAC CACAGGTGAC CCAAGCGGTC     180
   AGAGGTCCTT TCCCAGACCT CACCGACCCT CACTACACCT CTCCACCTTC GTGTCTGATG     240
   TTACGTCGAA AGTACAGGTC TGACTTGTAG TGGTTCCTGT TGAGGTTCGC GGTCCAAAAG     300
   AAATTTTACT TGTCAGATGT TCGACTACTG TGACGGTATA TGATGACACG GTTTAGCTAC     360
   TAATGCTGCC CGAAACAATA CCTGAGGACC CCAGTTCCTT GGAGTCAGTG GCAGAGGAGT     420
   CGGTTTTGCT GTGGGGGTAG ACAGATAGGT GACC                                 454
(2) INFORMATION FOR SEQ ID NO: 4:
   (i) SEQUENCE CHARACTERISTICS:
      (A) LENGTH: 364 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION:1..363
   (xi) SEQUENCE DESCRIPTION: SEQ ID NO: 4:
   GAC ATT CAG CTG ACC CAG TCT CCA TCC TCC TTT TCT GTT TCT CTA GGA     48
   Asp Ile Gln Leu Thr Gln Ser Pro Ser Ser Phe Ser Val Ser Leu Gly
               155                     160                     165
   GAC AGA GTC ACC ATT ACT TGC AAG GCA AGT GAG GAC ATA TAT AAT CGG     96
   Asp Arg Val Thr Ile Thr Cys Lys Ala Ser Glu Asp Ile Tyr Asn Arg
           170                     175                     180
   TTA ACC TGG TAT CAG CAG AAA CCA GGA AAT GCT CCT AGG CTC TTA ATA    144
   Leu Thr Trp Tyr Gln Gln Lys Pro Gly Asn Ala Pro Arg Leu Leu Ile
       185                     190                     195
   TCT GGT GCA ACC AGT TTG GAA ACT GGG GTT CCT TCA AGA TTC AGT GGC    192
   Ser Gly Ala Thr Ser Leu Glu Thr Gly Val Pro Ser Arg Phe Ser Gly
   200                     205                     210                     215
   AGT GGA TCT GGA AAG GAT TAC ACT CTC AGC ATT ACC AGT CTT CAG ACT    240
   Ser Gly Ser Gly Lys Asp Tyr Thr Leu Ser Ile Thr Ser Leu Gln Thr
                   220                     225                     230
   GAA GAT GTT GCT ACC TAT TAC TGT CAA CAG TAT TGG AGT AAT CCG TAC    288
   Glu Asp Val Ala Thr Tyr Tyr Cys Gln Gln Tyr Trp Ser Asn Pro Tyr
               235                     240                     245
   ACG TTC GGA GGG GGG ACC AAG CTG GAA ATA AGA CGG GCT GAT GCT GCA    336
   Thr Phe Gly Gly Gly Thr Lys Leu Glu Ile Arg Arg Ala Asp Ala Ala
           250                     255                     260
   CCA ACT GTA TCC ATC TTC CCA CCA TCC A                              364
   Pro Thr Val Ser Ile Phe Pro Pro Ser
       265                     270
(2) INFORMATION FOR SEQ ID NO: 5:
   (i) SEQUENCE CHARACTERISTICS:
      (A) LENGTH: 121 amino acids
      (B) TYPE: amino acid
      (D) TOPOLOGY: linear
   (ii) MOLECULE TYPE: protein
   (xi) SEQUENCE DESCRIPTION: SEQ ID NO: 5:
   Asp Ile Gln Leu Thr Gln Ser Pro Ser Ser Phe Ser Val Ser Leu Gly
     1               5                  10                  15
   Asp Arg Val Thr Ile Thr Cys Lys Ala Ser Glu Asp Ile Tyr Asn Arg
                20                  25                  30
   Leu Thr Trp Tyr Gln Gln Lys Pro Gly Asn Ala Pro Arg Leu Leu Ile
            35                  40                  45
   Ser Gly Ala Thr Ser Leu Glu Thr Gly Val Pro Ser Arg Phe Ser Gly
        50                  55                  60
   Ser Gly Ser Gly Lys Asp Tyr Thr Leu Ser Ile Thr Ser Leu Gln Thr
    65                  70                  75                  80
   Glu Asp Val Ala Thr Tyr Tyr Cys Gln Gln Tyr Trp Ser Asn Pro Tyr
                    85                  90                  95
   Thr Phe Gly Gly Gly Thr Lys Leu Glu Ile Arg Arg Ala Asp Ala Ala
               100                 105                 110
   Pro Thr Val Ser Ile Phe Pro Pro Ser
           115                 120
(2) INFORMATION FOR SEQ ID NO: 6:
   (i) SEQUENCE CHARACTERISTICS:
      (A) LENGTH: 364 base pairs
      (B) TYPE: nucleic acid
      (C) STRANDEDNESS: double
      (D) TOPOLOGY: linear
   (iv) ANTI-SENSE: YES
   (xi) SEQUENCE DESCRIPTION: SEQ ID NO: 6:
   CTGTAAGTCG ACTGGGTCAG AGGTAGGAGG AAAAGACAAA GAGATCCTCT GTCTCAGTGG    60
   TAATGAACGT TCCGTTCACT CCTGTATATA TTAGCCAATT GGACCATAGT CGTCTTTGGT   120
   CCTTTACGAG GATCCGAGAA TTATAGACCA CGTTGGTCAA ACCTTTGACC CCAAGGAAGT   180
   TCTAAGTCAC CGTCACCTAG ACCTTTCCTA ATGTGAGAGT CGTAATGGTC AGAAGTCTGA   240
   CTTCTACAAC GATGGATAAT GACAGTTGTC ATAACCTCAT TAGGCATGTG CAAGCCTCCC   300
   CCCTGGTTCG ACCTTTATTC TGCCCGACTA CGACGTGGTT GACATAGGTA GAAGGGTGGT   360
   AGGT                                                                364
(2) INFORMATION FOR SEQ ID NO: 7:
   (i) SEQUENCE CHARACTERISTICS:
      (A) LENGTH: 746 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION:3..737
   (xi) SEQUENCE DESCRIPTION: SEQ ID NO: 7:
   AA GCT TCT CTA CAG TTA CTG AGC ACA CAG GAC CTC ACC ATG GGA TGG        47
      Ala Ser Leu Gln Leu Leu Ser Thr Gln Asp Leu Thr Met Gly Trp
                  125                 130                 135
   AGC TGT ATC ATC CTC TTC TTG GTA GCA ACA GCT ACA GGT GTC CAC TCC       95
   Ser Cys Ile Ile Leu Phe Leu Val Ala Thr Ala Thr Gly Val His Ser
               140                 145                 150
   GAC ATC CAG ATG ACC CAG AGC CCA AGC AGC CTG AGC GCC AGC GTG GGT      143
   Asp Ile Gln Met Thr Gln Ser Pro Ser Ser Leu Ser Ala Ser Val Gly
           155                 160                 165
   GAC AGA GTG ACC ATC ACC TGT AAG GCA AGT GAG GAC ATA TAT AAT CGG      191
   Asp Arg Val Thr Ile Thr Cys Lys Ala Ser Glu Asp Ile Tyr Asn Arg
       170                 175                 180
   TTA ACC TGG TAC CAG CAG AAG CCA GGT AAG GCT CCA AAG CTG CTG ATC        239
   Leu Thr Trp Tyr Gln Gln Lys Pro Gly Lys Ala Pro Lys Leu Leu Ile
   185                 190                 195                 200
   TCT GGT GCA ACC AGT TTG GAA ACT GGT GTG CCA AGC AGA TTC AGC GGT        287
   Ser Gly Ala Thr Ser Leu Glu Thr Gly Val Pro Ser Arg Phe Ser Gly
                   205                 210                 215
   AGC GGT AGC GGT ACC GAC TTC ACC TTC ACC ATC AGC AGC CTC CAG CCA        335
   Ser Gly Ser Gly Thr Asp Phe Thr Phe Thr Ile Ser Ser Leu Gln Pro
               220                 225                 230
   GAG GAC ATC GCC ACC TAC TAC TGC CAA CAG TAT TGG AGT AAT CCG TAC        383
   Glu Asp Ile Ala Thr Tyr Tyr Cys Gln Gln Tyr Trp Ser Asn Pro Tyr
           235                 240                 245
   ACG TTC GGC CAA GGG ACC AAG GTG GAA ATC AAA CGA ACT GTG GCT GCA        431
   Thr Phe Gly Gln Gly Thr Lys Val Glu Ile Lys Arg Thr Val Ala Ala
       250                 255                 260
   CCA TCT GTC TTC ATC TTC CCG CCA TCT GAT GAG CAG TTG AAA TCT GGA        479
   Pro Ser Val Phe Ile Phe Pro Pro Ser Asp Glu Gln Leu Lys Ser Gly
   265                 270                 275                 280
   ACT GCC TCT GTT GTG TGC CTG CTG AAT AAC TTC TAT CCC AGA GAG GCC        527
   Thr Ala Ser Val Val Cys Leu Leu Asn Asn Phe Tyr Pro Arg Glu Ala
                   285                 290                 295
   AAA GTA CAG TGG AAG GTG GAT AAC GCC CTC CAA TCG GGT AAC TCC CAG        575
   Lys Val Gln Trp Lys Val Asp Asn Ala Leu Gln Ser Gly Asn Ser Gln
               300                 305                 310
   GAG AGT GTC ACA GAG CAG GAC AGC AAG GAC AGC ACC TAC AGC CTC AGC        623
   Glu Ser Val Thr Glu Gln Asp Ser Lys Asp Ser Thr Tyr Ser Leu Ser
           315                 320                 325
   AGC ACC CTG ACG CTG AGC AAA GCA GAC TAC GAG AAA CAC AAA GTC TAC 671
   Ser Thr Leu Thr Leu Ser Lys Ala Asp Tyr Glu Lys His Lys Val Tyr
   330 335 340
   GCC TGC GAA GTC ACC CAT CAG GGC CTG AGC TCG CCC GTC ACA AAG AGC 719
   Ala Cys Glu Val Thr His Gln Gly Leu Ser Ser Pro Val Thr Lys Ser
   345 350 355 360
   TTC AAC AGG GGA GAG TGT TAGAAGCTT 746
   Phe Asn Arg Gly Glu Cys
   365
(2) INFORMATION FOR SEQ ID NO: 8:
   (i) SEQUENCE CHARACTERISTICS:
      (A) LENGTH: 245 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Ala Ser Leu Gln Leu Leu Ser Thr Gln Asp Leu Thr Met Gly Trp Ser
   1 5 10 15
   Cys Ile Ile Leu Phe Leu Val Ala Thr Ala Thr Gly Val His Ser Asp
   20 25 30
   Ile Gln Met Thr Gln Ser Pro Ser Ser Leu Ser Ala Ser Val Gly Asp
   35 40 45
   Arg Val Thr Ile Thr Cys Lys Ala Ser Glu Asp Ile Tyr Asn Arg Leu
   50 55 60
   Thr Trp Tyr Gln Gln Lys Pro Gly Lys Ala Pro Lys Leu Leu Ile Ser
   65 70 75 80
   Gly Ala Thr Ser Leu Glu Thr Gly Val Pro Ser Arg Phe Ser Gly Ser
   85 90 95
   Gly Ser Gly Thr Asp Phe Thr Phe Thr Ile Ser Ser Leu Gln Pro Glu
   100 105 110
   Asp Ile Ala Thr Tyr Tyr Cys Gln Gln Tyr Trp Ser Asn Pro Tyr Thr
   115 120 125
   Phe Gly Gln Gly Thr Lys Val Glu Ile Lys Arg Thr Val Ala Ala Pro
   130 135 140
   Ser Val Phe Ile Phe Pro Pro Ser Asp Glu Gln Leu Lys Ser Gly Thr
   145 150 155 160
   Ala Ser Val Val Cys Leu Leu Asn Asn Phe Tyr Pro Arg Glu Ala Lys
   165 170 175
   Val Gln Trp Lys Val Asp Asn Ala Leu Gln Ser Gly Asn Ser Gln Glu
   180 185 190
   Ser Val Thr Glu Gln Asp Ser Lys Asp Ser Thr Tyr Ser Leu Ser Ser
   195 200 205
   Thr Leu Thr Leu Ser Lys Ala Asp Tyr Glu Lys His Lys Val Tyr Ala
   210 215 220
   Cys Glu Val Thr His Gln Gly Leu Ser Ser Pro Val Thr Lys Ser Phe
   225 230 235 240
   Asn Arg Gly Glu Cys
   245
(2) INFORMATION FOR SEQ ID NO: 9:
   (i) SEQUENCE CHARACTERISTICS:
      (A) LENGTH: 746 base pairs
      (B) TYPE: nucleic acid
      (C) STRANDEDNESS: double
      (D) TOPOLOGY: linear
   (iv) ANTI-SENSE: YES
   (xi) SEQUENCE DESCRIPTION: SEQ ID NO: 9:
   TTCGAAGAGA TGTCAATGAC TCGTGTGTCC TGGAGTGGTA CCCTACCTCG ACATAGTAGG    60
   AGAAGAACCA TCGTTGTCGA TGTCCACAGG TGAGGCTGTA GGTCTACTGG GTCTCGGGTT   120
   CGTCGGACTC GCGGTCGCAC CCACTGTCTC ACTGGTAGTG GACATTCCGT TCACTCCTGT   180
   ATATATTAGC CAATTGGACC ATGGTCGTCT TCGGTCCATT CCGAGGTTTC GACGACTAGA   240
   GACCACGTTG GTCAAACCTT TGACCACACG GTTCGTCTAA GTCGCCATCG CCATCGCCAT   300
   GGCTGAAGTG GAAGTGGTAG TCGTCGGAGG TCGGTCTCCT GTAGCGGTGG ATGATGACGG   360
   TTGTCATAAC CTCATTAGGC ATGTGCAAGC CGGTTCCCTG GTTCCACCTT TAGTTTGCTT   420
   GACACCGACG TGGTAGACAG AAGTAGAAGG GCGGTAGACT ACTCGTCAAC TTTAGACCTT   480
   GACGGAGACA ACACACGGAC GACTTATTGA AGATAGGGTC TCTCCGGTTT CATGTCACCT   540
   TCCACCTATT GCGGGAGGTT AGCCCATTGA GGGTCCTCTC ACAGTGTCTC GTCCTGTCGT   600
   TCCTGTCGTG GATGTCGGAG TCGTCGTGGG ACTGCGACTC GTTTCGTCTG ATGCTCTTTG   660
   TGTTTCAGAT GCGGACGCTT CAGTGGGTAG TCCCGGACTC GAGCGGGCAG TGTTTCTCGA   720
   AGTTGTCCCC TCTCACAATC TTCGAA                                        746
(2) INFORMATION FOR SEQ ID NO: 10:
   (i) SEQUENCE CHARACTERISTICS:
      (A) LENGTH: 436 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION:3..14
   (ix) FEATURE:
      (A) NAME/KEY: CDS
      (B) LOCATION:18..434
   (xi) SEQUENCE DESCRIPTION: SEQ ID NO: 10:
   AA GCT TTA CAG TTA CNC AGC ACA CAG GAC CTC ACC ATG GGA TGG AGC        47
      Ala Leu Gln Leu     Ser Thr Gln Asp Leu Thr Met Gly Trp Ser
                            1               5                  10
   TGT ATC ATC CTC TTC TTG GTA GCA ACA GCT ACA GGT GTC CAC TCC CAG       95
   Cys Ile Ile Leu Phe Leu Val Ala Thr Ala Thr Gly Val His Ser Gln
                    15                  20                  25
   GTC CAA CTG CAG GAG AGC GGT CCA GGT CTT GTG AGA CCT AGC CAG ACC      143
   Val Gln Leu Gln Glu Ser Gly Pro Gly Leu Val Arg Pro Ser Gln Thr
               30                  35                  40
   CTG AGC CTG ACC TGC ACC GTG TCT GGC TTT TCG TTA ACC AGT TAT GGT      191
   Leu Ser Leu Thr Cys Thr Val Ser Gly Phe Ser Leu Thr Ser Tyr Gly
           45                  50                  55
   GTC CAC TGG GTG AGA CAG CCA CCT GGA CGA GGT CTT GAG TGG ATT GGA      239
   Val His Trp Val Arg Gln Pro Pro Gly Arg Gly Leu Glu Trp Ile Gly
       60                  65                  70
   GTG ATG TGG AGA GGT GGA AGC ACA GAC TAC AAT GCA GCT TTC ATG TCC      287
   Val Met Trp Arg Gly Gly Ser Thr Asp Tyr Asn Ala Ala Phe Met Ser
   75                  80                  85                  90
   AGA CTG AAC ATC ACC AAG GAC AAC AGC AAG AAC CAG GTG AGC TTA AGA      335
   Arg Leu Asn Ile Thr Lys Asp Asn Ser Lys Asn Gln Val Ser Leu Arg
                   95                 100                 105
   CTC AGC AGC GTG ACA GCC GCC GAC ACC GCG GTC TAT TAT TGT GCA AAA      383
   Leu Ser Ser Val Thr Ala Ala Asp Thr Ala Val Tyr Tyr Cys Ala Lys
               110                 115                 120
   TCC ATG ATA ACG ACC GGC TTC GTG ATG GAC TCC TGG GGT CAA GGC TCA      431
   Ser Met Ile Thr Thr Gly Phe Val Met Asp Ser Trp Gly Gln Gly Ser
           125                 130                 135
   CTA GT                                                               436
   Leu
(2) INFORMATION FOR SEQ ID NO: 11:
   (i) SEQUENCE CHARACTERISTICS:
      (A) LENGTH: 4 amino acids
      (B) TYPE: amino acid
      (D) TOPOLOGY: linear
   (ii) MOLECULE TYPE: protein
   (xi) SEQUENCE DESCRIPTION: SEQ ID NO: 11:
   Ala Leu Gln Leu
   1
(2) INFORMATION FOR SEQ ID NO: 12:
   (i) SEQUENCE CHARACTERISTICS:
      (A) LENGTH: 139 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Ser Thr Gln Asp Leu Thr Met Gly Trp Ser Cys Ile Ile Leu Phe Leu
   1 5 10 15
   Val Ala Thr Ala Thr Gly Val His Ser Gln Val Gln Leu Gln Glu Ser
   20 25 30
   Gly Pro Gly Leu Val Arg Pro Ser Gln Thr Leu Ser Leu Thr Cys Thr
   35 40 45
   Val Ser Gly Phe Ser Leu Thr Ser Tyr Gly Val His Trp Val Arg Gln
   50 55 60
   Pro Pro Gly Arg Gly Leu Glu Trp Ile Gly Val Met Trp Arg Gly Gly
   65 70 75 80
   Ser Thr Asp Tyr Asn Ala Ala Phe Met Ser Arg Leu Asn Ile Thr Lys
   85 90 95
   Asp Asn Ser Lys Asn Gln Val Ser Leu Arg Leu Ser Ser Val Thr Ala
   100 105 110
   Ala Asp Thr Ala Val Tyr Tyr Cys Ala Lys Ser Met Ile Thr Thr Gly
   115 120 125
   Phe Val Met Asp Ser Trp Gly Gln Gly Ser Leu
   130 135
(2) INFORMATION FOR SEQ ID NO: 13:
   (i) SEQUENCE CHARACTERISTICS:
      (A) LENGTH: 436 base pairs
      (B) TYPE: nucleic acid
      (C) STRANDEDNESS: double
      (D) TOPOLOGY: linear
   (iv) ANTI-SENSE: YES
   (xi) SEQUENCE DESCRIPTION: SEQ ID NO: 13:
   TTCGAAATGT CAATGNGTCG TGTGTCCTGG AGTGGTACCC TACCTCGACA TAGTAGGAGA 60
   AGAACCATCG TTGTCGATGT CCACAGGTGA GGGTCCAGGT TGACGTCCTC TCGCCAGGTC 120
   CAGAACACTC TGGATCGGTC TGGGACTCGG ACTGGACGTG GCACAGACCG AAAAGCAATT 180
   GGTCAATACC ACAGGTGACC CACTCTGTCG GTGGACCTGC TCCAGAACTC ACCTAACCTC 240
   ACTACACCTC TCCACCTTCG TGTCTGATGT TACGTCGAAA GTACAGGTCT GACTTGTAGT 300
   GGTTCCTGTT GTCGTTCTTG GTCCACTCGA ATTCTGAGTC GTCGCACTGT CGGCGGCTGT 360
   GGCGCCAGAT AATAACACGT TTTAGGTACT ATTGCTGGCC GAAGCACTAC CTGAGGACCC 420
   CAGTTCCGAG TGATCA 436
(2) INFORMATION FOR SEQ ID NO: 14:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO: 14:
   GACATTCAGC TGACCCAGTC TCCA 24
(2) INFORMATION FOR SEQ ID NO: 15:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO: 15:
   GATCAAGCTT GACATTCAGC TGACCCAGTC TCCA 34
(2) INFORMATION FOR SEQ ID NO: 16:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (xi) SEQUENCE DESCRIPTION: SEQ ID NO: 16:
   ACTAGTCGAC CATCCTCCTT TTCTGTTTCT CTAGGAG 37
(2) INFORMATION FOR SEQ ID NO: 17:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 17:
   GATCAAGCTT CTCTACAGTT ACTGAGCACA 30
(2) INFORMATION FOR SEQ ID NO: 18:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 18:
   CCGATTATAT ATGTCCTCAC TTGCCTTACA GGTGATGGTC AC 42
(2) INFORMATION FOR SEQ ID NO: 19:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (xi) SEQUENCE DESCRIPTION: SEQ ID NO: 19:
   AGTGAGGACA TATATAATCG GTTAACCTGG TACCAGCAGA AG 42
(2) INFORMATION FOR SEQ ID NO: 20:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 20:
   AGTTTCCAAA CTGGTTGCAC CAGAGATCAG CAGCTTTGG 39
(2) INFORMATION FOR SEQ ID NO: 21:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 21:
   GGTGCAACCA GTTTGGAAAC TGGTGTGCCA AGCAGA 36
(2) INFORMATION FOR SEQ ID NO: 22:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 22:
   GTACGGATTA CTCCAATACT GTTGGCAGTA GTAGGTGGC 39
(2) INFORMATION FOR SEQ ID NO: 23:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 23:
   CAGTATTGGA GTAATCCGTA CACGTTCGGC CAAGGGACC 39
(2) INFORMATION FOR SEQ ID NO: 24:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 24:
   GATCAAGCTT CTAACACTCT CCCCTGTTGA 30
(2) INFORMATION FOR SEQ ID NO: 25:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 25:
   GATCAAGCTT TACAGTTACT CAGCACACAG 30
(2) INFORMATION FOR SEQ ID NO: 26:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 26:
   GTGGACACCA TAACTGGTGA AGGTGAAGCC 30
(2) INFORMATION FOR SEQ ID NO: 27:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO: 27:
   AGTTATGGTG TCCACTGGGT GAGACAGCCA 30
(2) INFORMATION FOR SEQ ID NO: 28:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (xi) SEQUENCE DESCRIPTION: SEQ ID NO: 28:
   TTGTAGTCTG TGCTTCCACC TCTCCACATC ACTCCAATCC ACTCAAG 47
(2) INFORMATION FOR SEQ ID NO: 29:
   (i) SEQUENCE CHARACTERISTICS:
      (A) LENGTH: 51 base pairs
      (B) TYPE: nucleic acid
      (C) STRANDEDNESS: single
      (D) TOPOLOGY: linear
   (xi) SEQUENCE DESCRIPTION: SEQ ID NO: 29:
   GGAGTCCATC ACGAAGCCGG TCGTTATCAT GGATTTTGCA CAATAATAGA C 51
(2) INFORMATION FOR SEQ ID NO: 30:
   (i) SEQUENCE CHARACTERISTICS:
      (A) LENGTH: 72 base pairs
      (B) TYPE: nucleic acid
      (C) STRANDEDNESS: single
      (D) TOPOLOGY: linear
   (xi) SEQUENCE DESCRIPTION: SEQ ID NO: 30:
   AAATCCATGA TAACGACCGG CTTCGTGATG GACTCCTGGG GTCAAGGCTC ACTAGTCACA 60
   GTCTCCTCAG CC 72
(2) INFORMATION FOR SEQ ID NO: 31:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 31:
   TAGAGTCCTG AGGGAATTCG GACAGCCGGG AAGGTG 36
(2) INFORMATION FOR SEQ ID NO: 32:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 32:
   GTTGTCCTTG GTGATGTTCA GTCTGGACAT GAAAGCTGC 39
(2) INFORMATION FOR SEQ ID NO: 33:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO: 33:
   CTGAACATCA CCAAGGACAA CAGCAAGAAC CAGTTCAGC 39
(2) INFORMATION FOR SEQ ID NO: 34:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 34:
   ACTGGTTAAC GAAAAGCCAG ACACGGTGCA GGTCAG 36
(2) INFORMATION FOR SEQ ID NO: 35:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 35:
   GGCTTTTCGT TAACCAGTTA TGGTGTCCAC TGGGTG 36
(2) INFORMATION FOR SEQ ID NO: 36:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 36:
   AAATTGCCGT TTCGAAGTGT CTACCAGCAT TGTCAC 36
(2) INFORMATION FOR SEQ ID NO: 37:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 37:
   AAATTGCCGT TTCGAATTGT CCTTGGTGAT GTTCAG 36
(2) INFORMATION FOR SEQ ID NO: 38:
   (i) SEQUENCE CHARACTERISTICS:
      (A) LENGTH: 34 base pairs
      (B) TYPE: nucleic acid
      (C) STRANDEDNESS: single
      (D) TOPOLOGY: linear
   (xi) SEQUENCE DESCRIPTION: SEQ ID NO: 38:
   TTCGAAACGG CAATTTAGCT TGAGACTCAG CAGC 34
(2) INFORMATION FOR SEQ ID NO: 39:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (xi) SEQUENCE DESCRIPTION: SEQ ID NO: 39:
   GAAGCACAGA CTACAATGCA GCTTTCATGT CCAGAGTGAC AATGCTG 47
(2) INFORMATION FOR SEQ ID NO: 40:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (xi) SEQUENCE DESCRIPTION: SEQ ID NO: 40:
   GATCAAGCTT TACAGTTACT CAGCACAG 28
(2) INFORMATION FOR SEQ ID NO: 41:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 41:
   ACTGGTTAAC GAAAAGCCAG ACACGGTGCA GGTCAG 36
(2) INFORMATION FOR SEQ ID NO: 42:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 42:
   GGCTTTTCGT TAACCAGTTA TGGTGTCCAC TGGGTG 36
(2) INFORMATION FOR SEQ ID NO: 43:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO: 43:
   TAGAGTCCTG AGGGAATTCG GACAGCCGGG AAGGTG 36
(2) INFORMATION FOR SEQ ID NO: 44:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO: 44:
   AACCAGGTGA GCTTAAGACT CAGCAGCGTG ACA 33
(2) INFORMATION FOR SEQ ID NO: 45:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO: 45:
   TCTTAAGCTC ACCTGGTTCT TGCTGTTGTC CTT 33
(2) INFORMATION FOR SEQ ID NO: 46:
   (i) SEQUENCE CHARACTERISTICS:
      (A) LENGTH: 5 amino acids
      (B) TYPE: amino acid
      (C) STRANDEDNESS: single
      (D) TOPOLOGY: linear
   (xi) SEQUENCE DESCRIPTION: SEQ ID NO: 46:
   Ser Tyr Gly Val His
   1 5